(19) 

Europäisches
Patentamt
European
Patent Office
Office européen
des brevets

(11) **EP 4 717 714 A1**

(12) **EUROPEAN PATENT APPLICATION**
published in accordance with Art. 153(4) EPC

(43) Date of publication:
**01.04.2026 Bulletin 2026/14**

(21) Application number: **24810411.9**

(22) Date of filing: **22.05.2024**

(51) International Patent Classification (IPC):
*C07K 16/46* (2006.01)     *C12N 15/13* (2006.01)
*G01N 33/68* (2006.01)     *A61K 39/395* (2006.01)
*A61P 35/00* (2006.01)     *A61P 35/02* (2006.01)

(52) Cooperative Patent Classification (CPC):
A61K 39/395; A61P 35/00; A61P 35/02;
C07K 16/00; C07K 16/46; G01N 33/68

(86) International application number:
**PCT/CN2024/094648**

(87) International publication number:
**WO 2024/240171 (28.11.2024 Gazette 2024/48)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **22.05.2023 CN 202310575606**

(71) Applicant: **Shanghai Junshi Biosciences Co., Ltd.**
**Shanghai 201203 (CN)**

(72) Inventors:
• **WANG, Jing**
**Shanghai 201203 (CN)**
• **HONG, Min**
**Shanghai 201203 (CN)**
• **ZHOU, Yuehua**
**Shanghai 201203 (CN)**
• **WU, Chun**
**Shanghai 201203 (CN)**

• **LIU, Dandan**
**Shanghai 201203 (CN)**
• **XIE, Qiuju**
**Shanghai 201203 (CN)**
• **DU, Xiaojie**
**Shanghai 201203 (CN)**
• **LIU, Chenchen**
**Shanghai 201203 (CN)**
• **FENG, Hui**
**Shanghai 201203 (CN)**
• **YAO, Sheng**
**Shanghai 201203 (CN)**

(74) Representative: **E. Blum & Co. AG**
**Franklinturm**
**Hofwiesenstrasse 349**
**8050 Zürich (CH)**

Remarks:
The complete document including Reference
Table(s) and the Sequence Listing(s) can be
downloaded from the EPO website

(54) **ANTI-PD-1-ANTI-VEGF BISPECIFIC ANTIBODY, PHARMACEUTICAL COMPOSITION THEREOF AND USE THEREOF**

(57)     The present application provides a bispecific antibody targeting PD-1 and VEGF, and a composition comprising same. Also provided are a nucleic acid molecule encoding the bispecific antibody of the present application, a vector and a host cell for expressing the bispecific antibody of the present application, and therapeutic and diagnostic methods using the antibody or antigen-binding fragment thereof of the present application and the use thereof.

Figure 14

**EP 4 717 714 A1**

## Description

The present application claims priority to the Chinese patent application no. 2023105756067 titled "ANTI-PD-1-ANTI-VEGF BISPECIFIC ANTIBODY, PHARMACEUTICAL COMPOSITION THEREOF AND USE THEREOF" and filed with the China Patent Office on May 22, 2023, the entire contents of which are incorporated in present application by reference.

Technical Field

The present application relates to the field of tumor therapy and immunological biology, and in particular to an anti-PD-1-anti-VEGF bispecific antibody, a pharmaceutical composition thereof and the use thereof.

Background Art

Vascular endothelial growth factor (VEGF) is a type of growth factor that can promote the division and proliferation of endothelial cells, promote the formation of new blood vessels, and improve vascular permeability. It binds to vascular endothelial growth factor receptor on the cell surface and functions by activating tyrosine kinase signal transduction pathway. In tumor tissues, tumor cells, tumor-invading macrophages and mast cells can secrete high levels of VEGF, stimulate tumor vascular endothelial cells in paracrine form, promote the proliferation and migration of endothelial cells, induce angiogenesis, promote the continuous growth of tumors, improve vascular permeability, cause fibrin deposition in surrounding tissues, promote the infiltration of monocytes, fibroblasts and endothelial cells, and facilitate the formation of tumor stroma and the entry of tumor cells into new blood vessels, thereby promoting tumor metastasis. Consequently, inhibiting tumor angiogenesis is currently considered one of the most promising methods for treating a tumor. The VEGF family comprises: VEGFA, VEGFB, VEGFC, VEGFD, and placental growth factor (PIGF). The VEGF family molecules have different affinities for several receptors. VEGFA functions primarily via binding to VEGFR1, VEGFR2 and NRP-1. Currently, there are a variety of humanized monoclonal antibodies targeting human VEGF, especially VEGFA, such as bevacizumab, which was successively approved by the United States Food and Drug Administration in 2004 for the treatment of non-small cell lung cancer, renal cell carcinoma, cervical cancer, metastatic colorectal cancer and other tumors.

Programmed cell death receptor-1 (PD-1), also known as CD279, is a type I transmembrane glycoprotein membrane surface receptor, which belongs to the CD28 immunoglobulin superfamily and is commonly expressed in T cells, B cells and myeloid cells. PD-1 has two natural ligands, i.e., PD-L1 and PD-L2. Both PD-L1 and PD-L2 belong to the B7 superfamily and are constitutively or inductively expressed on the surface of a variety of cell membranes, including non-hematopoietic cells and a variety of tumor cells. PD-L1 is mainly expressed in T cells, B cells, dendritic cells (DC cells), microvascular endothelial cells and a variety of tumor cells. PD-L2 is expressed only in antigen presenting cells such as dendritic cells and macrophages. The interaction between PD-1 and its ligand can inhibit lymphatic activation, T cell proliferation and the secretion of cytokines such as IL-2 and IFN-$\gamma$. Due to the broad-spectrum anti-tumor prospects and amazing efficacy of PD-1 antibodies, the industry generally believes that antibodies against the PD-1 pathway will bring breakthrough progress in the treatment of various tumors, for example, in the treatment of non-small cell lung cancer, renal cell carcinoma, ovarian cancer, melanoma (Homet M.B., Parisi G., et al., Anti-PD-1 Therapy in Melanoma. SeminOncol. 2015 Jun; 42(3): 466-473), lymphoma, and anemia (Held SA, Heine A, et al., Advancesin immunotherapy of chronic myeloid leukemia CML. Curr Cancer Drug Targets. 2013 Sep; 13(7): 768-74).

Bifunctional antibody, also known as bispecific antibody, is specific drugs that target two different antigens simultaneously. It can be produced by immunosorting and purification, or can be obtained by genetic engineering. A variety of bispecific antibodies currently developed are in the form of IgG-ScFv, namely the Morrison mode (1997 Coloma MJ, Morrison SL. Design and production of novel tetravalent bispecific antibodies. Nat Biotechnol. Nature Biotechnology, 1997; 15: 159-163), which has been shown to be an ideal existence form for bifunctional antibodies due to its advantages in antibody engineering, expression and purification similar to the naturally occurring IgG form (Miller BR, Demarest SJ, et al., Stability engineering of scFvs for the development of bispecific and multivalent antibodies. Protein Eng Des Sel 2010; 23: 549-57; Fitzgerald J, Lugovskoy A.Rational engineering of antibody therapeutics targeting multiple oncogene pathways. MAbs 2011; 3: 299-309). Currently, there is a need to develop a bifunctional antibody drug that targets both PD-1 and VEGF (e.g., VEGFA) simultaneously.

Summary

The object of the present application is to provide an anti-PD-1-anti-VEGF bispecific antibody, a pharmaceutical composition thereof and use thereof, wherein the bispecific antibody is capable of simultaneously binding VEGF and PD-1, blocking binding of VEGFA to VEGFR2 and blocking binding of PD-1 to PD-L1.

A first aspect of the present application provides a bispecific antibody comprising a first protein functional region and a second protein functional region; wherein: the first protein functional region is an anti-PD-1 antibody or an antigen-binding fragment thereof, and the second protein functional region is an anti-VEGF antibody or an antigen-binding fragment thereof, wherein the anti-VEGF antibody or the antigen-binding fragment thereof comprises a heavy chain variable region comprising HCDR1, HCDR2, and HCDR3 with amino acid sequences as set forth in SEQ ID NO: 7, SEQ ID NO: 8, and SEQ ID NO: 9, respectively.

In some embodiments of the present application, the anti-PD-1 antibody or the antigen-binding fragment thereof comprises a heavy chain variable region and/or a light chain variable region, wherein the amino acid sequences of HCDR1, HCDR2, and HCDR3 comprised in the heavy chain variable region are selected from the group consisting of the amino acid sequences of HCDR1, HCDR2, and HCDR3 comprised in the heavy chain variable region of nivolumab, pembrolizumab, cemiplimab, toripalimab, sintilimab, or camrelizumab, respectively; the amino acid sequences of LCDR1, LCDR2, and LCDR3 comprised in the light chain variable region are selected from the group consisting of the amino acid sequences of LCDR1, LCDR2, and LCDR3 comprised in the light chain variable region of nivolumab, pembrolizumab, cemiplimab, toripalimab, sintilimab, or camrelizumab, respectively.

In some embodiments of the present application, the anti-PD-1 antibody or the antigen-binding fragment thereof comprises a heavy chain variable region comprising HCDR1, HCDR2 and HCDR3 with amino acid sequences as set forth in SEQ ID NO: 1, SEQ ID NO: 2 and SEQ ID NO: 3, respectively, and/or a light chain variable region comprising LCDR1, LCDR2 and LCDR3 with amino acid sequences as set forth in SEQ ID NO: 4, SEQ ID NO: 5 and SEQ ID NO: 6, respectively.

In some embodiments of the present application, the anti-PD-1 antibody or the antigen-binding fragment thereof comprises: a heavy chain variable region having at least 90%, 91%, 92%, 93%, 94%, 95%, 96%, 97%, 98%, 99%, or 100% sequence identity to an amino acid sequence as set forth in SEQ ID NO: 10, and a light chain variable region having at least 90%, 91%, 92%, 93%, 94%, 95%, 96%, 97%, 98%, 99%, or 100% sequence identity to an amino acid sequence as set forth in SEQ ID NO: 11; and/or the anti-VEGF antibody or the antigen-binding fragment thereof comprises: a heavy chain variable region having at least 90%, 91%, 92%, 93%, 94%, 95%, 96%, 97%, 98%, 99% or 100% sequence identity to an amino acid sequence as set forth in SEQ ID NO: 12.

In some embodiments of the present application, the anti-PD-1 antibody or the antigen-binding fragment thereof is selected from the group consisting of Fab, Fab', F(ab')2, Fd, Fv, dAb, complementary determining region fragment, single-domain antibody, single chain antibody, humanized antibody, chimeric antibody and diabody; and/or the anti-VEGF antibody or the antigen-binding fragment thereof is selected from the group consisting of Fab, Fab', F(ab')2, Fd, Fv, dAb, complementary determining region fragment, single-domain antibody, single chain antibody, humanized antibody, chimeric antibody and diabody.

In some embodiments of the present application, the first protein functional region is an immunoglobulin, and/or the second protein functional region is a single-domain antibody.

In some embodiments of the present application, the first protein functional region is an immunoglobulin, wherein the immunoglobulin is selected from the group consisting of IgG, IgA, IgD, IgE and IgM; preferably IgG.

In some embodiments of the present application, the first protein functional region is an immunoglobulin, wherein the constant region of the immunoglobulin is derived from a human antibody; preferably, the constant region of the immunoglobulin is selected from the group consisting of a constant region of human IgG1, IgG2, IgG3 or IgG4.

In some embodiments of the present application, the first protein functional region is an immunoglobulin, and the second protein functional region is a single-domain antibody; the single-domain antibodies are two in number, and one terminus of each of the single-domain antibodies is respectively connected to the C-terminus or N-terminus of two heavy chains of the immunoglobulin; or one terminus of each of the single-domain antibodies is respectively connected to the C-terminus or N-terminus of two light chains of the immunoglobulin; or the N-terminus of each of the single-domain antibodies is respectively connected to the C-terminus of the heavy chain constant region CH1 of the immunoglobulin, and the C-terminus of each of the single-domain antibodies is respectively connected to the N-terminus of the heavy chain constant region CH2 of the immunoglobulin.

In some embodiments of the present application, the bispecific antibody is selected from the group consisting of the following forms: (1) the first polypeptide chain of the bispecific antibody is represented by a formula VLPD-1-CL, and the second polypeptide chain is represented by a formula VHVEGF-linker-VHPD-1-CH1-hinge-CH2-CH3; (2) the first polypeptide chain of the bispecific antibody is represented by the formula VLPD-1-CL, and the second polypeptide chain is represented by the formula VHPD-1-CH1-hinge-CH2-CH3-linker-VHVEGF; (3) the first polypeptide chain of the bispecific antibody is represented by the formula VLPD-1-CL, and the second polypeptide chain is represented by the formula VHPD-1-CH1-linker-VHVEGF-linker-hinge-CH2-CH3; and (4) the first polypeptide chain of the bispecific antibody is represented by the formula VHVEGF-linker-VLPD-1-CL, and the second polypeptide chain is represented by the formula VHPD-1-CH1-hinge-CH2-CH3.

In some embodiments of the present application, the bispecific antibody is selected from the group consisting of the following: (1) the first polypeptide chain comprises an amino acid sequence as set forth in SEQ ID NO: 13, and the second polypeptide chain comprises an amino acid sequence as set forth in SEQ ID NO: 14; (2) the first polypeptide chain comprises an amino acid sequence as set forth in SEQ ID NO: 13, and the second polypeptide chain comprises an amino acid sequence as set forth in SEQ ID NO: 15; (3) the first polypeptide chain comprises an amino acid sequence as set forth in SEQ ID NO: 13, and the second polypeptide chain comprises an amino acid sequence as set forth in SEQ ID NO: 16; and (4) the first polypeptide chain comprises an amino acid sequence as set forth in SEQ ID NO: 17, and the second polypeptide chain comprises an amino acid sequence as set forth in SEQ ID NO: 18.

A second aspect of the present application provides an isolated nucleic acid molecule encoding the bispecific antibody according to the first aspect of the present application.

A third aspect of the present application provides a vector comprising the isolated nucleic acid molecule according to the second aspect of the present application.

A fourth aspect of the present application provides a host cell comprising the isolated nucleic acid molecule according to the second aspect of the present application, or the vector according to the third aspect of the present application.

A fifth aspect of the present application provides a method of preparing the bispecific antibody according to the first aspect of the present application, comprising the steps of culturing the host cell according to the fourth aspect of the present application, and obtaining the bispecific antibody from the culture.

A sixth aspect of the present application provides a conjugate comprising the bispecific antibody according to the first aspect of the present application and a conjugation moiety, wherein the conjugation moiety is a therapeutically active substance or a detectable label; preferably, the therapeutically active substance is a cytotoxin, a cytokine or a radioactive nuclide; preferably, the detectable label is selected from the group consisting of a radioisotope, a fluorescent substance, a luminescent substance, a chromogenic substance, and an enzyme.

A seventh aspect of the present application provides a kit comprising the bispecific antibody according to the first aspect of the present application, or comprising the conjugate according to the sixth aspect of the present application; preferably, the kit further comprises a second antibody capable of specifically binding to the bispecific antibody; preferably, the second antibody further comprises a detectable label, more preferably, the detectable label is selected from the group consisting of a radioisotope, a fluorescent substance, a luminescent substance, a chromogenic substance and an enzyme.

An eighth aspect of the present application provides the use of the bispecific antibody according to the first aspect of the present application in the preparation of a kit; preferably, the kit is used for detecting the presence or level of VEGF and/or PD-1 in a sample.

A ninth aspect of the present application provides a pharmaceutical composition comprising the bispecific antibody according to the first aspect of the present application, or comprising the conjugate according to the sixth aspect of the present application; preferably, the pharmaceutical composition further comprises a pharmaceutically acceptable excipient.

A tenth aspect of the present application provides the use of the bispecific antibody according to the first aspect of the present application, or the conjugate according to the sixth aspect of the present application, or the pharmaceutical composition according to the ninth aspect of the present application in the preparation of a drug for preventing and/or treating malignant tumors.

An eleventh aspect of the present application provides a method for treating or ameliorating a malignant tumor, comprising administering to a patient in need thereof the bispecific antibody according to the first aspect of the present application, or the conjugate according to the sixth aspect of the present application, or the pharmaceutical composition according to the ninth aspect of the present application; the present application also provides the bispecific antibody according to the first aspect, or the conjugate according to the sixth aspect of the present application, or the pharmaceutical composition according to the ninth aspect of the present application for use in treating or ameliorating a malignant tumor; wherein the malignant tumor is a PD-L1 and/or VEGF overexpressing tumor; preferably, the malignant tumor is a solid tumor; more preferably, the malignant tumor is lung cancer, colon cancer, colorectal cancer (CRC), melanoma or ovarian cancer; more preferably, the lung cancer is non-small cell lung cancer (NSCLC) or small cell lung cancer (SCLC); more preferably, the non-small cell lung cancer is squamous non-small cell lung cancer or non-squamous non-small cell lung cancer (nsq-NSCLC); more preferably, the non-squamous non-small cell lung cancer is locally advanced or metastatic nsq-NSCLC.

A twelfth aspect of the present application provides a method of modulating an immune response and modulating tumor angiogenesis, comprising administering to a patient in need thereof the bispecific antibody according to the first aspect of the present application, or the conjugate according to the sixth aspect of the present application, or the pharmaceutical composition according to the ninth aspect of the present application; preferably, the modulating the immune response is achieved by blocking the binding of PD-1 to a receptor thereof, and the modulating tumor angiogenesis is achieved by blocking the binding of VEGF to a receptor thereof.

A thirteenth aspect of the present application provides the use of the bispecific antibody according to the first aspect of the present application, or the conjugate according to the sixth aspect of the present application, or the pharmaceutical composition according to the ninth aspect of the present application in the preparation of the following drug or reagent: a reagent for detecting the level of VEGF in a sample.

A fourteenth aspect of the present application provides an in vitro method for non-diagnostic purpose and non-therapeutic purpose, comprising the step of administering to a cell an effective amount of the bispecific antibody according to the first aspect of the present application, or the conjugate according to the sixth aspect of the present application, or the pharmaceutical composition according to the ninth aspect of the present application, wherein the method is selected from the group consisting of the following:

(1) a method for detecting the level of VEGF in a sample, a method for blocking the binding of VEGF to VEGFR, a method for down-regulating the activity or level of VEGF, a method for abolishing the stimulating effect of VEGF on the proliferation of vascular endothelial cells, a method for inhibiting the proliferation of vascular endothelial cells, and a method for blocking tumor angiogenesis; and/or
(2) a method for blocking the binding of PD-1 to PD-L1, a method for down-regulating the activity or level of PD-1, a method for abolishing the immunosuppression of PD-1 on the body, a method for promoting the secretion of IFN-γ in T lymphocytes, and a method for promoting the secretion of IL-2 in T lymphocytes.

Beneficial effects of the present application:
the bispecific antibody provided by the present application has good thermal stability and long-term stability, can specifically bind to VEGFA very well, can block the binding of VEGFA to VEGFR2 very effectively, and specifically abolish the immunosuppression of VEGFA on the body and the promotion of angiogenesis; can specifically bind to PD-1 very well, and can block the binding of PD-1 and PD-L1 very effectively, specifically abolish the immunosuppression of PD-1 on the body, activate the immune response, and have a good effect of inhibiting tumor proliferation.

Of course, practicing any of the products or methods of the present application does not necessarily need to achieve all of the advantages described above simultaneously.

Brief Description of the Drawings

In order to more clearly illustrate the examples of the present application or the technical solutions in the prior art, the drawings used in the description of the examples or the prior art will be briefly introduced below. It is obvious that the drawings in the following description are only some examples of the present application, and other examples can be obtained by those skilled in the art according to these drawings.

FIG. 1 is a schematic structural diagram of DotAb4;
FIG. 2a is a schematic structural diagram of antibody 23; FIG. 2b is a schematic structural diagram of antibody 24; FIG. 2c is a schematic structural diagram of antibody 27; FIG. 2d is a schematic structural diagram of antibody 28;
FIG. 3a is a graph showing the association and dissociation curves of antibody JS001 to human PD-1; FIG. 3b is a graph showing the association and dissociation curves of antibody 23 to human PD-1; FIG. 3c is a graph showing the association and dissociation curves of antibody 24 to human PD-1; FIG. 3d is a graph showing the association and dissociation curves of antibody 27 to human PD-1; FIG. 3e is a graph showing the association and dissociation curves of antibody 28 to human PD-1;
FIG. 4a is a graph showing the association and dissociation curves of antibody DotAb4 to human VEGFA; FIG. 4b is a graph showing the association and dissociation curves of antibody 23 to human VEGFA; FIG. 4c is a graph showing the association and dissociation curves of antibody 24 to human VEGFA; FIG. 4d is a graph showing the association and dissociation curves of antibody 27 to human VEGFA; FIG. 4e is a graph showing the association and dissociation curves of antibody 28 to human VEGFA;
FIG. 5a is a graph showing the results of the binding of the antibody 27 to two antigens, human PD-1 and human VEGFA, simultaneously in the sample group to be tested; FIG. 5b is a graph showing the results of non-specific binding of human VEGFA to human PD-1 in the negative control group; FIG. 5c is a graph showing the results of non-specific binding of human VEGFA to JS001 in the negative control group;
FIG. 6a is a graph showing ELISA curves of the binding of antibody 23 and antibody 24 to human PD-1; FIG. 6b is a graph showing ELISA curves of the binding of antibody 27 and antibody 28 to human PD-1;
FIG. 7a is a graph showing ELISA curves of the binding of antibody 23 and antibody 24 to human VEGFA; FIG. 7b is a graph showing ELISA curves of the binding of antibody 27 and antibody 28 to human VEGFA;
FIG. 8a is a graph showing competitive inhibition ELISA curves of antibody 23 and antibody 24 blocking the binding of human VEGFR2 to VEGFA; FIG. 8b is a graph showing competitive inhibition ELISA curves of antibody 27 and antibody 28 blocking the binding of human VEGFR2 to VEGFA;
FIG. 9a is a graph showing competitive inhibition ELISA curves of antibody 23 and antibody 24 blocking the binding of human PD-L1 to PD-1; FIG. 9b is a graph showing competitive inhibition ELISA curves of antibody 27 and antibody 28 blocking the binding of human PD-L1 to PD-1;

FIG. 10a is a graph showing the results of activity detection of antibody 23 and antibody 24 in PD-1/PD-L1 luciferase reporter gene system; FIG. 10b is a graph showing the results of activity detection of antibody 27 and antibody 28 in PD-1/PD-L1 luciferase reporter gene system;

FIG. 11a is a graph showing the results of activity detection in a luciferase reporter gene system in which antibody 23 and antibody 24 block VEGF-induced NFAT signal; FIG. 11b is a graph showing the results of activity detection in a luciferase reporter gene system in which antibody 27 and antibody 28 block VEGF-induced NFAT signal;

FIG. 12a is a graph showing experimental results of antibody 23 and antibody 24 inhibiting VEGFA-induced proliferation of HUVEC cells; FIG. 12b is a graph showing experimental results of antibody 27 and antibody 28 inhibiting VEGFA-induced proliferation of HUVEC cells;

FIG. 13a is a graph showing experimental results of antibodies promoting the secretion of cytokine IL2 in donor 1 mixed lymphocyte reaction; FIG. 13b is a graph showing experimental results of antibodies promoting the secretion of cytokine IL2 in donor 2 mixed lymphocyte reaction; FIG. 13c is a graph showing experimental results of antibodies promoting the secretion of cytokine IFN-$\gamma$ in donor 1 mixed lymphocyte reaction; FIG. 13d is a graph showing experimental results of antibodies promoting the secretion of cytokine IFN-$\gamma$ in donor 2 mixed lymphocyte reaction;

FIG. 14 is a graph showing the inhibitory effect of a test substance on a B-PD-1 humanized mouse model transplanted with MC38 tumor;

FIG. 15 is a graph showing the inhibitory effect of a test substance on an NDG mouse model transplanted with A375 tumor.

TAB001 is JS001 in the drawings of the present application.

Detailed Description of Embodiments

The following examples illustrate the present application but are not intended to limit the present application in any way. The present application has been described in detail herein, and specific embodiments of the present application are also disclosed herein. Various changes and improvements to the specific embodiments of the present application would be obvious to a person skilled in the art without departing from the spirit and scope of the present application.

In the present application, unless otherwise specified, the scientific and technical terms used in the present application have the meanings commonly understood by those skilled in the art. In addition, the cell culture, molecular genetics, nucleic acid chemistry, and immunology laboratory operation steps used in the present application are conventional steps that are widely used in the corresponding art. Meanwhile, to better understand the present application, the definitions and explanations of the relevant terms are provided below.

VEGF of the present application specifically refers to VEGFA. The anti-VEGF antibody of the present application specifically binds to VEGFA and is capable of blocking the binding of VEGFA to VEGFR2 very effectively. In some embodiments, VEGFA can be VEGFA from a primate (rhesus monkey, cynomolgus monkey, or human), and in some embodiments, VEGFA may be a mouse VEGFA.

As used in the present application, when referring to the amino acid sequence of the VEGFA protein (GenBank ID: NP_001165097.1), it includes the full length of the VEGFA protein, as well as the fusion proteins of VEGFA, such as fragments fused to an Fc protein fragment (mFc or hFc) of mouse or human IgG. However, one skilled in the art understands that mutations or variations (including but not limited to substitutions, deletions and/or additions) can be naturally occurred or artificially introduced into the amino acid sequence of the VEGFA protein without affecting its biological function. Accordingly, in the present application, the term "VEGFA protein" should include all such sequences, including natural or artificial variants thereof. In addition, when a sequence fragment of the VEGFA protein is described, it further includes the corresponding sequence fragment in a natural or artificial variant of the VEGFA protein. In one embodiment of the present application, the amino acid sequence of the VEGFA protein is as set forth in SEQ ID NO: 19 (excluding the terminal His, the VEGFA protein used in the examples of the present application is purchased from ARCO, name: Human VEGF165 Protein, His Tag, catalog number: VE5-H5248, lot no.: 2410-2069F1-146).

As used in the present application, when referring to the amino acid sequence of the VEGFR2 protein (KDR) (GenBank ID: NP_002244), it includes the full length of the VEGFR2 protein, or the extracellular fragment of VEGFR2, i.e., VEGFR2-ECD, or a fragment comprising VEGFR2-ECD, and further includes the fusion proteins of VEGFR2-ECD, such as fragments fused to an Fc protein fragment (mFc or hFc) of mouse or human IgG. However, one skilled in the art understands that mutations or variations (including but not limited to substitutions, deletions and/or additions) can be naturally occurred or artificially introduced into the amino acid sequence of the VEGFR2 protein without affecting its biological function. Accordingly, in the present application, the term "VEGFR2 protein" should include all such sequences, including natural or artificial variants thereof. In addition, when a sequence fragment of the VEGFR2 protein is described, it further includes the corresponding sequence fragment in a natural or artificial variant of the VEGFR2 protein. In one embodiment of the present application, the amino acid sequence of VEGFR2 is as set forth in SEQ ID NO: 20 (the VEGFR2 protein used in the examples of the present application is purchased from Sino Biological, Inc., and is named: VEGFR2/KDR Protein, Human, Recombinant (His Tag), Biotinylated, Cat:10012-H08H-B).

As used in the present application, the VEGFR is VEGFR1 and/or VEGFR2 unless otherwise specified; the specific protein sequence thereof is a sequence known in the prior art, and reference can be made to the sequences disclosed in the prior literature or GenBank. For example VEGFR1 (VEGFR1, NCBI Gene ID: 2321); VEGFR2(VEGFR2, NCBI Gene ID:3791).

As used in the present application, when referring to the amino acid sequence of the PD-1 protein (Programmed cell death protein 1, NCBI GenBank: NM_005018), it includes the full length of the PD-1 protein, or the extracellular fragment of PD-1, PD-1ECD, or a fragment comprising PD-1ECD, and further includes the fusion proteins of PD-1ECD, such as fragments fused to an Fc protein fragment (mFc or hFc) of mouse or human IgG. However, one skilled in the art understands that mutations or variations (including but not limited to substitutions, deletions and/or additions) can be naturally occurred or artificially introduced into the amino acid sequence of the PD-1 protein without affecting its biological function. Accordingly, in the present application, the term "PD-1 protein" should include all such sequences, including natural or artificial variants thereof. In addition, when a sequence fragment of the PD-1 protein is described, it further includes the corresponding sequence fragment in a natural or artificial variant of the PD-1 protein.

As used in the present application, the term $EC_{50}$ refers to the concentration for 50% of maximum effect, which means the concentration that can cause 50% of maximum effect.

As used in the present application, the term "antibody" refers to an immunoglobulin molecule generally composed of two pairs of polypeptide chains, each pair having a "light" (L) chain and a "heavy" (H) chain. In a general sense, the heavy chain can be understood as a polypeptide chain with a larger molecular weight in an antibody, and the light chain refers to a polypeptide chain with a smaller molecular weight in an antibody. Light chains can be classified as kappa and lambda light chains. Heavy chains can be generally classified into $\mu, \delta, \gamma, \alpha$ or $\varepsilon$, and the isotypes of antibodies are defined as IgM, IgD, IgG, IgA and IgE, respectively. Within the light chain and heavy chain, the variable region and constant region are generally linked by a "J" region of about 12 or more amino acids, and the heavy chain further comprises a "D" region of about 3 or more amino acids. Each heavy chain consists of a heavy chain variable region (VH) and a heavy chain constant region (CH). The heavy chain constant region consists of 3 domains (CH1, CH2 and CH3). Each light chain consists of a light chain variable region (VL) and a light chain constant region (CL). The light chain constant region consists of one domain CL. The constant region of the antibody can mediate the binding of the immunoglobulin to a host tissue or factor (comprising various cells (e.g., effector cells) of the immune system and the first component of the classical complement system (C1q)). The VH region and VL region can also be subdivided into regions with high variability called complementary determining region (CDR), among which more conservative regions called framework regions (FRs) are interspersed. Individual VHs and VLs consist of 3 CDRs and 4 FRs arranged from amino terminal to carboxyl terminal, in the following order: FR1, CDR1, FR2, CDR2, FR3, CDR3, FR4. The variable regions (VH and VL) of each heavy chain/light chain pair form antibody binding sites respectively. The assignment of amino acids in each region or domain follows the definitions of Kabat Sequences of Proteins of ImmunologicalInterest (National Institutes of Health, Bethesda, Md. (1987 and 1991)) or Chothia & Lesk (1987) J. Mol. Biol. 196: 901-917; Chothia et al. (1989) Nature 342: 878-883. In particular, the heavy chain may also comprise more than 3 CDRs, such as 6, 9 or 12. For example, in the bifunctional antibody of the present application, the heavy chain may be an IgG antibody heavy chain C-terminally linked to a scFv of another antibody, in which case the heavy chain contains 9 CDRs. The term "antibody" is not limited by any particular method of producing antibodies. For example, it includes, in particular, recombinant antibodies, monoclonal antibodies, and polyclonal antibodies. Antibodies may be antibodies of different isotypes, for example, IgG (e.g., IgG1, IgG2, IgG3, or IgG4 subtypes), IgA1, IgA2, IgD, IgE, or IgM antibodies.

As used in the present application, the term "antigen-binding fragment" of an antibody refers to a polypeptide comprising a fragment of a full-length antibody that retains the ability to specifically bind to the same antigen to which the full-length antibody binds, and/or competes with the full-length antibody to specifically bind to the antigen, and is also referred to as "antigen-binding portion". Usually see, Fundamental Immunology, Ch. 7 (Paul, W., ed., 2nd ed., Raven Press, N.Y. (1989), which is incorporated herein by reference in its entirety for all purposes. Antigen-binding fragments of antibodies can be produced by recombinant DNA technology or by enzymatic or chemical cleavage of intact antibodies. In some instances, antigen-binding fragments comprise Fab, Fab', F(ab')2, Fd, Fv, single-domain antibodies (sdAbs) and complementary determining region (CDR) fragments, single chain antibodies (e.g., scFvs), chimeric antibodies, diabodies, and polypeptides comprising at least a portion of an antibody sufficient to confer specific antigen-binding ability to the polypeptides.

As used in the present application, the term "Fd fragment" means an antibody fragment consisting of the VH and CH1 domains; the term "Fv fragment" means an antibody fragment consisting of the VL and VH domains of the antibody's single arm; the term "dAb fragment" means an antibody fragment consisting of a VH domain (Ward et al., Nature 341: 544-546 (1989)); the term "Fab fragment" means an antibody fragment consisting of the VL, VH, CL and CH1 domains; the term "F(ab')2 fragment" means an antibody fragment comprising two Fab fragments linked by a disulfide bridge on the hinge region.

In some instances, an antigen-binding fragment of an antibody is a single chain antibody (e.g., scFv) in which the VL and VH domains are paired to form a monovalent molecule by a linker that enables the VL and VH domains to be produced as a single polypeptide chain (for example, see, Bird et al., Science 242: 423-426 (1988) and Huston et al., Proc. Natl. Acad. Sci. USA 85: 5879-5883 (1988)). Such scFv molecules can have a general structure: NH2-VL-linker-VH-COOH or NH2-VH-linker-VL-COOH. Suitable linkers in the prior art consist of repeated GGGGS amino acid sequences or variants thereof. For example, linkers having an amino acid sequence (GGGGS)4 can be used, but variants thereof can also be used (Holliger et al., (1993), Proc. Natl. Acad. Sci. USA 90: 6444-6448). Other linkers that can be used in the present application are described by Alfthan et al. (1995), Protein Eng. 8: 725-731, Choi et al. (2001), Eur. J. Immunol. 31: 94-106, Hu et al. (1996), Cancer Res. 56: 3055-3061, Kipriyanov et al. (1999), J. Mol. Biol. 293: 41-56, and Roovers et al. (2001), Cancer Immunol.

In some instances, an antigen-binding fragment of an antibody is a diabody, i.e., a bivalent antibody, wherein the VH and VL domains are expressed on a single polypeptide chain, but a linker used is too short to allow for pairing between two domains of the same chain, thereby forcing the domain to pair with the complementary domain of the other chain and producing two antigen binding sites (for example, see, Holliger P. et al., Proc. Natl. Acad. Sci. USA 90: 6444-6448 (1993), and Poljak R. J. et al., Structure 2: 1121-1123 (1994)).

Conventional techniques known to those skilled in the art (for example, recombinant DNA technology or enzymatic or chemical fragmentation) can be used to obtain from a given antibody the antigen-binding fragments of the antibody (for example, the above-mentioned antibody fragments) which can be screened for specificity in the same manner by which intact antibodies are screened.

In the present application, unless the context expressly indicates, when the term "antibody" is mentioned, it includes not only the complete antibody, but also the antigen-binding fragment of the antibody.

As used in the present application, the term "single-domain antibody (sdAb)", also referred to as "nanobody", "single domain antibody", "single variable domain on heavy chain antibody", "VHH", "nanobody" and "heavy chain antibody (HcAb)", has the meaning commonly understood by those skilled in the art, which refers to an antibody fragment consisting of a single monomeric variable antibody domain (e.g., a single heavy chain variable region), typically derived from the variable region of a heavy chain antibody (e.g., a camelid antibody or a shark antibody). Typically, nanobodies consist of 4 framework regions and 3 complementarity determining regions, and have the structure of FR1-CDR1-FR2-CDR2-FR3-CDR3-FR4. Nanobodies may be truncated at the N-terminus or C-terminus such that they comprise only a portion of FR1 and/or FR4, or lack one or both of those backbone regions, as long as they substantially retain antigen binding and specificity.

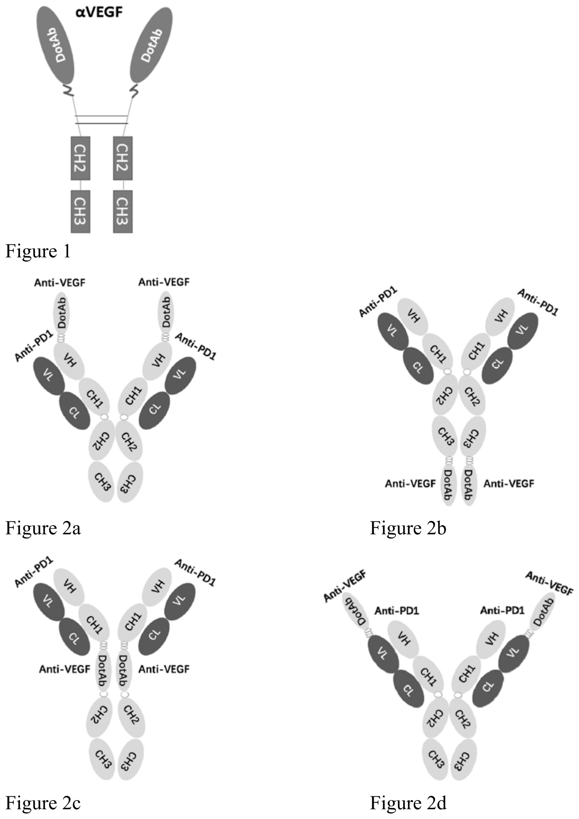

The DotAb described in the present application is a single-domain antibody that binds to VEGF (anti-VEGF), which can be referred to the single-domain antibody specifically binding to VEGFA described in patent CN 117242092 A, and in some embodiments, the DotAb comprises CDRs of the VEGFA-binding single-domain antibody described in CN 117242092 A, or CDRs derived from the VEGFA-binding single-domain antibody described in CN 117242092 A. In some embodiments, the DotAb comprises the CDR, FR and/or the complete amino acid sequence of a VEGFA-binding single-domain antibody selected from the group consisting of 16C2.1 and 21A5.1, such as HCDR1, HCDR2, and HCDR3 as set forth in SEQ ID NO: 7, SEQ ID NO: 8, and SEQ ID NO: 9 in the present application. In some embodiments, the DotAb is a single-domain antibody having a sequence as set forth in SEQ ID NO: 12 of the present application or a derived sequence thereof, wherein the derived sequence has at least 85%, 90%, 91%, 92%, 93%, 95%, 96%, 97%, 98%, 99% consistency or identity to the sequence as set forth in SEQ ID NO: 12 of the present application. DotAb4 is a single-domain antibody linked to an Fc, and is referred to as a heavy chain antibody. DotAb4 can comprise two identical polypeptide chains. The structure of each polypeptide chain is shown by the formula DotAb-hinge-CH2-CH3, and its schematic structural diagram is shown in FIG. 1.

As used in the present application, the terms "monoclonal Ab" and "monoclonal antibody" refer to an antibody or a fragment of an antibody from a population of highly homologous antibody molecules, that is, a population of completely identical antibody molecules except for natural mutation that may occur spontaneously. Monoclonal antibodies are highly specific for single epitopes on antigens. Polyclonal antibodies are relative to monoclonal antibodies and typically contain at least 2 or more different antibodies that typically recognize different epitopes on antigens. Monoclonal antibodies can generally be obtained using hybridoma techniques first reported by Kohler et al. (Nature, 256: 495, 1975), but can also be obtained using recombinant DNA techniques (such as see, U.S.P 4,816,567).

As used in the present application, the term "chimeric antibody" refers to an antibody in which a portion of the light chain or/and heavy chain of the antibody is derived from one antibody (which may be derived from a particular species or belong to a particular antibody class or subclass), and another portion of the light chain or/and the heavy chain is derived from another antibody (which may be derived from the same or a different species or belong to the same or a different antibody class or subclass), but which in any case retains the activity of binding to an antigen of interest (U.S.P 4,816,567 to Cabilly et al.; Morrison et al., Proc. Natl. Acad. Sci. USA, 81: 6851 6855(1984)).

As used in the present application, the term "humanized antibody" refers to an antibody or antibody fragment obtained by replacing all or part of the CDR region of a human immunoglobulin (recipient antibody) with a CDR region of a non-human antibody (donor antibody), wherein the donor antibody may be a non-human (e.g., mouse, rat, or rabbit) antibody having the intended specificity, affinity, or reactivity. In addition, some amino acid residues of the framework region (FR) of the recipient antibody can also be replaced with amino acid residues of the corresponding non-human antibody, or with amino acid residues of other antibodies, to further improve or optimize the performance of the antibody. More details on humanized antibodies can be found in, for example, Jones et al., Nature, 321: 522 525 (1986); Reichmann et al.,Nature,332:323 329(1988); Presta,Curr.Op.Struct.Biol.,2:593 596(1992); and Clark, Immunol. Today 21: 397 402 (2000).

As used in the present application, the term "epitope" refers to a site on an antigen to which an immunoglobulin or antibody specifically binds. "Epitope" is also referred to in the art as an "antigenic determinant". An epitope or an antigenic determinant generally consist of chemically active surface groups of molecules such as amino acids, carbohydrates or sugar side chains, and generally have specific three dimensional structural characteristics and specific charge characteristics. For example, an epitope generally includes at least 3, 4, 5, 6, 7, 8, 9, 10, 11, 12, 13, 14, or 15 contiguous or non-contiguous amino acids in a unique spatial conformation and can be "linear" or "conformational". See, for example, Epitope Mapping Protocols in Methods in Molecular Biology, Vol. 66, G. E. Morris, Ed. (1996). In a linear epitope, all interaction sites between a protein and an interacting molecule (such as an antibody) are present linearly along the primary amino acid sequence of the protein. In a conformational epitope, the interaction sites exist across amino acid residues in the protein that are spaced apart from each other.

As used in the present application, the term "isolated" or "separated" refers to a substance that has been obtained from its natural state through artificial means. If a certain "isolated" substance or component appears in nature, it may be possible that its natural environment has changed, or the substance has been isolated from the natural environment, or both. For example, a polynucleotide or polypeptide that is not isolated naturally exists in a living animal, and the same polynucleotide or polypeptide of high purity isolated from this natural state is said to be isolated. The term "isolated" or "separated" does not exclude the mixed artificial or synthetic substances, nor does it exclude the presence of other impure substances that do not affect the activity of the substance isolated.

As used in the present application, the term "vector" refers to a nucleic acid delivery vehicle in which polynucleotides can be inserted. When the vector enables a protein encoded by the inserted polynucleotide to be expressed, the vector is called an expression vector. Vectors can be transformed, transduced or transfected into host cells, and the genetic material elements carried by them can be expressed in host cells. Vectors are well known to those skilled in the art and include, but are not limited to: plasmid; phasmid; Cos plasmid; artificial chromosomes, such as yeast artificial chromosomes (YAC), bacterial artificial chromosomes (BAC) or P1-derived artificial chromosomes (PAC); bacteriophages such as lambda phage or M13 phage, animal viruses, etc. Animal viruses that may be used as vectors include, but are not limited to, retroviruses (including lentiviruses), adenoviruses, adeno-associated viruses, herpes viruses (such as herpes simplex virus), poxviruses, baculoviruses, papillomavirus, papovaviruses (such as SV40). A vector may contain a plurality of elements controlling expression, including but not limited to promoter sequences, transcription start sequences, enhancer sequences, selection elements, and reporter genes. In addition, the vector may also contain a replication initiation site.

As used in the present application, the term "host cell" refers to cells that can be used to introduce vectors, and includes but not limited to prokaryotic cells such as Escherichia coli or Bacillus subtilis, fungal cells such as yeast cells or Aspergillus, insect cells such as S2 Drosophila cells or Sf9 cells, or animal cells such as fibroblasts, CHO cells, COS cells, NSO cells, HeLa cells, BHK cells, HEK 293 cells or human cells.

As used in the present application, the term "specific binding" refers to a non-random binding reaction between two molecules, such as the reaction between an antibody and the antigen it targets. In certain embodiments, an antibody that specifically binds to an antigen (or is specific for an antigen) refers to an antibody that binds to the antigen with an affinity (KD) of less than about $10^{-5}$ M, for example, less than about $10^{-6}$ M, $10^{-7}$ M, $10^{-8}$ M, $10^{-9}$ M or $10^{-10}$ M or smaller. In some embodiments of the present application, the term "targeting" refers to specific binding.

As used in the present application, the term "KD" refers to the dissociation equilibrium constant of a particular antibody-antigen interaction, which is used to describe the binding affinity between an antibody and an antigen. The smaller the equilibrium dissociation constant, the tighter the antibody-antigen binding and the higher the affinity between the antibody and the antigen. Typically, an antibody binds to an antigen with a dissociation equilibrium constant (KD) of less than about $10^{-5}$ M, for example, less than about $10^{-6}$ M, $10^{-7}$ M, $10^{-8}$ M, $10^{-9}$ M or $10^{-10}$ M or smaller, for example, as determined in a BIACORE instrument using surface plasmon resonance (SPR).

As used in the present application, the terms "monoclonal antibody" and "monoclonal Ab" have the same meaning and are used interchangeably; the terms "polyclonal antibody" and "polyclonal Ab" have the same meaning and are used interchangeably; the terms "polypeptide" and "protein" have the same meaning and are used interchangeably. And in the present application, amino acids are typically denoted by one-letter and three-letter abbreviations known in the art. For example, alanine can be represented by A or Ala.

As used in the present application, the term "pharmaceutically acceptable excipient" refers to a carrier and/or excipient that is pharmacologically and/or physiologically compatible with the subject and the active ingredient and that is well known in the art (see, for example, Remington's Pharmaceutical Sciences. Edited by Gennaro AR, 19th ed. Pennsylvania: Mack Publishing Company, 1995), and includes but are not limited to: pH regulators, surfactants, adjuvants, and ionic strength enhancers. For example, the pH regulators include but are not limited to phosphate buffer; the surfactants include but are not limited to, cationic, anionic, or nonionic surfactants, such as Tween-80; the ion strength enhancers include but are not limited to sodium chloride.

As used in the present application, the term "adjuvant" refers to a non-specific immunoenhancer, which can enhance the immune response to an antigen or change the type of the immune response in a body, when delivered into the body with the antigen or delivered into the body in advance. There are a wide variety of adjuvants, including but not limited to, aluminum adjuvants (e.g., aluminum hydroxide), Freund's adjuvants (e.g., complete Freund's adjuvant, and incomplete Freund's adjuvant), Corynebacteriuum parvm, lipopolysaccharides, cytokines, etc. The Freund's adjuvants are currently the most commonly used adjuvants in animal experiments. the aluminium hydroxide adjuvants are widely used in clinical trials.

As used in the present application, the term "effective amount" refers to an amount sufficient to obtain, or at least partially obtain, the desired effect. For example, an effective amount for preventing a disease (e.g., a disease associated with binding of PD-1 to PD-L1 or overexpression of VEGF, such as a tumor) refers to an amount sufficient to prevent, stop, or delay the occurrence of a disease (e.g., a disease associated with binding of PD-1 to PD-L1 or overexpression of VEGF, such as a tumor); Therapeutically effective amount refers to an amount sufficient to cure or at least partially stop a disease and its complications in a patient who already has the disease. Determining such an effective amount is well within the capabilities of the skilled person. For example, the effective amount for therapeutic use will depend on the severity of the disease to be treated, the general state of the patient's own immune system, the general condition of the patient such as age, weight and sex, the manner in which the drug is administered, other treatments administered at the same time, etc.

Through intensive research and creative work, the inventors of the present application have obtained a humanized bispecific antibody capable of simultaneously binding VEGF and PD-1, and capable of simultaneously blocking the binding of VEGFA to VEGFR2 and blocking the binding of PD-1 to PD-L1.

The inventors of the present application have found that the bispecific antibody described in the present application not only has good thermal stability and long-term stability, but also can effectively bind PD-1 on the surface of human immune cells, abolish PD-L1 and PD-1-mediated immunosuppression, effectively activate the activity of T cells, and promote human immune cells to secrete cytokines IFN-$\gamma$ and IL-2; can specifically bind to VEGF antigen, block the binding of VEGFA to its receptor, thereby blocking the downstream signaling pathway, effectively inhibiting the proliferation of vascular endothelial cells induced by VEGFA, and inhibiting tumor-induced angiogenesis; can effectively inhibit the proliferation of HUVEC cells induced by VEGF, can be used for preparing drugs for preventing and treating malignant tumors, thereby inhibiting the development and deterioration of cancer.

The malignant tumors described in the present application are also referred to as cancers.

A first aspect of the present application provides a bispecific antibody comprising a first protein functional region and a second protein functional region; wherein: the first protein functional region is an anti-PD-1 antibody or an antigen-binding fragment thereof, and the second protein functional region is an anti-VEGF antibody or an antigen-binding fragment thereof, wherein the anti-VEGF antibody or the antigen-binding fragment thereof comprises a heavy chain variable region comprising HCDR1, HCDR2, and HCDR3 with amino acid sequences as set forth in SEQ ID NO: 7, SEQ ID NO: 8, and SEQ ID NO: 9, respectively.

In some embodiments of the present application, the anti-PD-1 antibody or the antigen-binding fragment thereof comprises a heavy chain variable region and/or a light chain variable region; the amino acid sequences of HCDR1, HCDR2, and HCDR3 comprised in the heavy chain variable region are selected from the group consisting of the amino acid sequences of HCDR1, HCDR2, and HCDR3 comprised in the heavy chain variable region of nivolumab, pembrolizumab, cemiplimab, toripalimab, sintilimab, or camrelizumab, respectively; the amino acid sequences of LCDR1, LCDR2, and LCDR3 comprised in the light chain variable region are selected from the group consisting of the amino acid sequences of LCDR1, LCDR2, and LCDR3 comprised in the light chain variable region of nivolumab, pembrolizumab, cemiplimab, toripalimab, sintilimab, or camrelizumab, respectively.

In some embodiments of the present application, the anti-PD-1 antibody or the antigen-binding fragment thereof comprises a heavy chain variable region comprising HCDR1, HCDR2 and HCDR3 with amino acid sequences as set forth in SEQ ID NO: 1, SEQ ID NO: 2 and SEQ ID NO: 3, respectively, and/or a light chain variable region comprising LCDR1, LCDR2 and LCDR3 with amino acid sequences as set forth in SEQ ID NO: 4, SEQ ID NO: 5 and SEQ ID NO: 6, respectively.

In some embodiments of the present application, the anti-PD-1 antibody or the antigen-binding fragment thereof comprises: a heavy chain variable region having at least 90%, 91%, 92%, 93%, 94%, 95%, 96%, 97%, 98%, 99%, or 100% sequence identity to an amino acid sequence as set forth in SEQ ID NO: 10, and a light chain variable region having at least 90%, 91%, 92%, 93%, 94%, 95%, 96%, 97%, 98%, 99%, or 100% sequence identity to an amino acid sequence as set forth in SEQ ID NO: 11; and/or the anti-VEGF antibody or the antigen-binding fragment thereof comprises: a heavy chain variable region having at least 90%, 91%, 92%, 93%, 94%, 95%, 96%, 97%, 98%, 99% or 100% sequence identity to an amino acid sequence as set forth in SEQ ID NO: 12.

The variable regions of the light and heavy chains determine antigen binding; the variable region of each chain contains three hypervariable regions, known as complementary determining regions (CDRs). The CDRs of the heavy chain (H) comprise HCDR1, HCDR2 and HCDR3, and the CDRs of the light chain (L) comprise LCDR1, LCDR2 and LCDR3; these CDRs are named by Kabat et al., see Sequences of Proteins of Immunological Interest, Fifth Edition (1991), volumes 1-3, NIH Publication 91-3242, Bethesda Md.

Methods and techniques for identifying CDRs in amino acid sequences of heavy chain variable regions and light chain variable regions are known in the art and can be used to identify CDRs in amino acid sequences of specific heavy chain variable regions and/or light chain variable regions disclosed in the present application. Exemplary well-known techniques that can be used to identify CDR boundaries include, for example, Kabat definition method, Chothia definition method, and AbM definition method. See, for example, Kabat, Sequences of Proteins of Immunological Interest, National Institutes of Health, Bethesda, Md. (1991); Al-Lazikani et al., Standard conformations for the canonical structures of immunoglobulins., J. Mol. Biol. 273: 927-948 (1997); and Martin AC et al., Modeling antibody hypervariable loops: a combined algorithm, Proc. Natl. Acid. Sci. USA 86: 9268-9272 (1989).

The amino acid sequences of the CDR regions of the bispecific antibody described in the present application (KABAT definition) are obtained by technical means well known to those skilled in the art, and the specific sequences are as follows:

three heavy chain complementary determining regions comprised in the anti-PD-1 antibody or the antigen-binding fragment thereof:

> PD-1-HCDR1: DYEMH (SEQ ID NO: 1);
> PD-1-HCDR2: VIESETGGTAYNQKFKG (SEQ ID NO: 2);
> PD-1-HCDR3: EGITTVATTYYWYFDV (SEQ ID NO: 3).

Three light chain complementary determining regions comprised in the anti-PD-1 antibody or the antigen-binding fragment thereof:

> PD-1-LCDR1: RSSQSIVHSNGNTYLE (SEQ ID NO: 4);
> PD-1-LCDR2: KVSNRFS (SEQ ID NO: 5);
> PD-1-LCDR3: FQGSHVPLT (SEQ ID NO: 6).

Three heavy chain complementary determining regions comprised in the anti-VEGF antibody or the antigen-binding fragment thereof:

> VEGF-HCDR1: ATDID (SEQ ID NO: 7);
> VEGF-HCDR2: RIFSPSDFTDYADSVKG (SEQ ID NO: 8);
> VEGF-HCDR3: PDTYAYNIFLDTPTYNALHY (SEQ ID NO: 9).

In some embodiments of the present application, the bispecific antibody, wherein

the anti-PD-1 antibody or the antigen-binding fragment thereof comprises a heavy chain variable region with an amino acid sequence as set forth in SEQ ID NO: 10, and a light chain variable region with an amino acid sequence as set forth in SEQ ID NO: 11; and/or

the anti-VEGF antibody or the antigen-binding fragment thereof comprises a heavy chain variable region with an amino acid sequence as set forth in SEQ ID NO: 12.

The heavy chain variable region (VH) comprised in the anti-PD-1 antibody or the antigen-binding fragment thereof, wherein the bold and underlined portions indicate the CDRs:

PD-1-VH:

QGQLVQSGAEVKKPGASVKVSCKASGYTFT**DYEMH**WVRQAPIHGLEWIG**VIESETGGT AYNQKFKG**RVTITADKSTSTAYMELSSLRSEDTAVYYCAR**EGITTVATTYYWYFDV**W GQGTTVTVSS (SEQ ID NO: 10).

The light chain variable region (VL) comprised in the anti-PD-1 antibody or the antigen-binding fragment thereof, wherein the bold and underlined portions indicate the CDRs:

PD-1-VL:

DVVMTQSPLSLPVTLGQPASISC**RSSQSIVHSNGNTYLE**WYLQKPGQSPQLLIY**KVSNRF**

**S**GVPDRFSGSGSGTDFTLKISRVEAEDVGVYYC**FQGSHVPLT**FGQGTKLEIK (SEQ ID

NO: 11).

The heavy chain variable region (VH) comprised in the anti-VEGF antibody or the antigen-binding fragment thereof, wherein the bold and underlined portions indicate the CDRs:
VEGF-VH:

EVQLVESGGGLVQPGGSLRLSCAISGFSLA**ATDID**WVRQAPGKGLEWVA**RIFSPSDFTD**

**YADSVKG**RFTISADTSKNTVYLQMNSLRAEDTAVYYCGR**PDTYAYNIFLDTPTYNALH**

**Y**RGQGTLVTVSS (SEQ ID NO: 12).

In some embodiments of the present application, the anti-PD-1 antibody or the antigen-binding fragment thereof is selected from the group consisting of Fab, Fab', F(ab')2, Fd, Fv, dAb, complementary determining region fragment, single-domain antibody, single chain antibody, humanized antibody, chimeric antibody and diabody; and/or the anti-VEGF antibody or the antigen-binding fragment thereof is selected from the group consisting of Fab, Fab', F(ab')2, Fd, Fv, dAb, complementary determining region fragment, single-domain antibody, single chain antibody, humanized antibody, chimeric antibody and diabody.

In some embodiments of the present application, the first protein functional region is an immunoglobulin, wherein the heavy chain variable region of the immunoglobulin comprises HCDR1, HCDR2 and HCDR3 with amino acid sequences as set forth in SEQ ID NO: 1, SEQ ID NO: 2 and SEQ ID NO: 3, respectively, and the light chain variable region of the immunoglobulin comprises LCDR1, LCDR2 and LCDR3 with amino acid sequences as set forth in SEQ ID NO: 4, SEQ ID NO: 5 and SEQ ID NO: 6, respectively; and/or the second protein functional region is a single-domain antibody, wherein the heavy chain variable region of the single-domain antibody comprises HCDR1, HCDR2 and HCDR3 with amino acid sequences as set forth in SEQ ID NO: 7, SEQ ID NO: 8 and SEQ ID NO: 9, respectively.

In some embodiments of the present application, the first protein functional region is an immunoglobulin, wherein the immunoglobulin is selected from the group consisting of IgG, IgA, IgD, IgE and IgM; preferably IgG.

In some embodiments of the present application, the first protein functional region is an immunoglobulin, wherein the immunoglobulin comprises a non-CDR region, and the non-CDR region is derived from a species that is not murine, such as from a human antibody.

In some embodiments of the present application, the first protein functional region is an immunoglobulin, wherein the constant region of the immunoglobulin is derived from a human antibody; preferably, the constant region of the immunoglobulin is selected from the group consisting of a constant region of human IgG1, IgG2, IgG3 or IgG4.

In some embodiments of the present application, the first protein functional region is an immunoglobulin, and the second protein functional region is a single-domain antibody; the single-domain antibodies are two in number, and one terminus of each of the single-domain antibodies is respectively connected to the C-terminus or N-terminus of two heavy chains of the immunoglobulin; or one terminus of each of the single-domain antibodies is respectively connected to the C-terminus or N-terminus of two light chains of the immunoglobulin; or the N-terminus of each of the single-domain antibodies is respectively connected to the C-terminus of the heavy chain constant region CH1 of the immunoglobulin, and the C-terminus of each of the single-domain antibodies is respectively connected to the N-terminus of the heavy chain constant region CH2 of the immunoglobulin.

In some embodiments of the present application, the bispecific antibody is selected from the group consisting of the following forms:

(1) the first polypeptide chain of the bispecific antibody is represented by a formula VLPD-1-CL, and the second polypeptide chain is represented by a formula VHVEGF-linker-VHPD-1-CH1-hinge-CH2-CH3;
(2) the first polypeptide chain of the bispecific antibody is represented by a formula VLPD-1-CL, and the second polypeptide chain is represented by a formula VHPD-1-CH1-hinge-CH2-CH3-linker-VHVEGF;
(3) the first polypeptide chain of the bispecific antibody is represented by a formula VLPD-1-CL, and the second polypeptide chain is represented by a formula VHPD-1-CH1-linker-VHVEGF-linker-hinge-CH2-CH3; and
(4) the first polypeptide chain of the bispecific antibody is represented by a formula VHVEGF-linker-VLPD-1-CL, and the second polypeptide chain is represented by a formula VHPD-1-CH1-hinge-CH2-CH3.

VLPD-1 as described in the present application refers to the VL of an anti-PD-1 antibody or an antigen-binding fragment thereof, and VHPD-1 refers to the VH of an anti-PD-1 antibody or an antigen-binding fragment thereof; VHVEGF refers to the VH of an anti-VEGF antibody or an antigen-binding fragment thereof.

The present application has no particular limitation on the amino acid sequence of the linker, as long as the purpose of the disclosure of the present application can be achieved. For example, the amino acid sequence of the linker may be (GGGGS)n, wherein n is selected from the group consisting of 1, 2, 3, 4 and 5, preferably 1, 2 and 3. The present application has no particular limitation on the amino acid sequence of the hinge, as long as the purpose of the disclosure of the present application can be achieved.

In some embodiments of the present application, the bispecific antibody is selected from the group consisting of the following:

(1) the first polypeptide chain comprises an amino acid sequence as set forth in SEQ ID NO: 13, and the second polypeptide chain comprises an amino acid sequence as set forth in SEQ ID NO: 14;
(2) the first polypeptide chain comprises an amino acid sequence as set forth in SEQ ID NO: 13, and the second polypeptide chain comprises an amino acid sequence as set forth in SEQ ID NO: 15;
(3) the first polypeptide chain comprises an amino acid sequence as set forth in SEQ ID NO: 13, and the second polypeptide chain comprises an amino acid sequence as set forth in SEQ ID NO: 16; and
(4) the first polypeptide chain comprises an amino acid sequence as set forth in SEQ ID NO: 17, and the second polypeptide chain comprises an amino acid sequence as set forth in SEQ ID NO: 18.

A second aspect of the present application provides an isolated nucleic acid molecule encoding the bispecific antibody according to the first aspect of the present application.

A third aspect of the present application provides a vector comprising the isolated nucleic acid molecule according to the second aspect of the present application.

A fourth aspect of the present application provides a host cell comprising the isolated nucleic acid molecule according to the second aspect of the present application, or comprising the vector according to the third aspect of the present application.

A fifth aspect of the present application provides a method of preparing the bispecific antibody according to the first aspect of the present application, comprising the steps of culturing the host cell according to the fourth aspect of the present application under suitable conditions, and recovering the bispecific antibody from the cell culture.

A sixth aspect of the present application provides a conjugate comprising the bispecific antibody according to the first aspect of the present application and a conjugation moiety, wherein the conjugation moiety is a therapeutically active substance or a detectable label, etc. In some embodiments, the conjugation moiety is selected from the group consisting of a toxin (such as a cytotoxin), a cytokine and a radioactive nuclide; in some embodiments, the conjugation moiety is selected from the group consisting of topoisomerase I inhibitor, camptothecin, and derivatives thereof. In some embodiments, the conjugation moiety is selected from the group consisting of a radioisotope, a fluorescent substance, a luminescent substance, a chromogenic substance, and an enzyme.

A seventh aspect of the present application provides a kit comprising the bispecific antibody according to the first aspect of the present application, or comprising the conjugate according to the sixth aspect of the present application.

Preferably, the kit further comprises a second antibody capable of specifically binding to the bispecific antibody; optionally, the second antibody further comprises a detectable label, such as a radioisotope, a fluorescent substance, a luminescent substance, a chromogenic substance or an enzyme.

An eighth aspect of the present application provides the use of the bispecific antibody according to the first aspect of the present application in the preparation of a kit; preferably, the kit is used for detecting the presence or level of VEGF and/or PD-1 in a sample.

A ninth aspect of the present application provides a pharmaceutical composition comprising the bispecific antibody according to the first aspect of the present application, or comprising the conjugate according to the sixth aspect of the present application; optionally, the pharmaceutical composition further comprises a pharmaceutically acceptable excipient.

The bispecific antibody provided in the first aspect of the present application or the pharmaceutical composition provided in the ninth aspect of the present application can be formulated into any dosage form known in the pharmaceutical art, for example, tablets, pills, suspensions, emulsions, solutions, gels, capsules, powders, granules, elixirs, lozenges, suppositories, injections (including injection solutions, sterile powders for injection and concentrated solutions for injection), inhalants, sprays, etc. The preferred dosage form depends on the intended mode of administration and therapeutic use. The pharmaceutical compositions of the present application should be sterile and stable under production and storage conditions, preferably the dosage form is an injection. Such an injection may be a sterile injection solution. For example, the sterile injectable solution can be prepared by the following methods; incorporating the bispecific antibody of the present application at a necessary dose into a suitable solvent, and optionally, incorporating other expected ingredients (including, but not limited to, a pH regulator, a surfactant, an adjuvant, an ionic strength enhancer, an isotonic agent, a preservative, a diluent, or any combination thereof), followed by filtration and sterilization. In addition, the sterile injection solution may be prepared as a sterile lyophilized powder (e.g., by vacuum drying or freeze drying) for easy storage and use. Such a sterile lyophilized powder can be dispersed in a suitable carrier, such as water for injection, before use.

In addition, the bispecific antibody of the present application may be present in a unit dose form in a pharmaceutical composition to facilitate administration. In certain embodiments, the unit dose is at least 1 mg, at least 5 mg, at least 10 mg, at least 15 mg, at least 25 mg, at least 30 mg, at least 45 mg, at least 50 mg, at least 75 mg, or at least 100 mg. Where the pharmaceutical composition is in a liquid (e.g., injection) dosage form, it can comprise the bispecific antibody of the present application at a concentration of at least 0.1 mg/mL, such as at least 0.25 mg/mL, at least 0.5 mg/mL, at least 1 mg/mL, at least 2.5 mg/mL, at least 5 mg/mL, at least 8 mg/mL, at least 10 mg/mL, at least 15 mg/mL, at least 25 mg/mL, at least 50 mg/mL, at least 75 mg/mL, or at least 100 mg/mL.

The bispecific antibody or pharmaceutical composition of the present application can be administered by any suitable method known in the art, including, but not limited to, oral, buccal, sublingual, ocular, topical, parenteral, rectal, intrathecal, intracytoplasmic reticulum, inguinal, intravesical, topical (e.g., powder, ointment or drops), or nasal route. However, for many therapeutic uses, the preferred route/mode of administration is parenteral administration (e.g., intravenous injection, subcutaneous injection, intraperitoneal injection, intramuscular injection). The skilled person should understand that the route and/or manner of administration will change according to the intended purpose. In a preferred embodiment, the bispecific antibody or pharmaceutical composition of the present application is administered by intravenous infusion or injection.

The bispecific antibodies or pharmaceutical compositions provided in the present application can be used alone or in combination, and can also be used in combination with additional pharmaceutically active agents (e.g., chemotherapeutic drugs for tumors). Such additional pharmaceutically active agents can be administered before, simultaneously with, or after administration of the bispecific antibody of the present application or the pharmaceutical composition of the present application.

In the present application, the dosing regimen may be adjusted to obtain the best desired response (e.g., therapeutic or prophylactic response). For example, a single administration may be performed, multiple administrations may be performed over a period of time or the dose may be proportionally decreased or increased as indicated by the urgency of the therapeutic situation.

In the prior art, the combination of anti-angiogenic therapy and immune checkpoint inhibitors has demonstrated good efficacy in many tumors. The combination of anti-VEGF antibodies, such as bevacizumab, and anti-PD-1/PD-L1 antibodies, such as nivolumab, pembrolizumab, atezolizumab, etc., has demonstrated good efficacy in ovarian cancer (Joyce F. Liu et al., JAMA Oncol. 2019; 5(12): 1731-1738.), non-small cell lung cancer (including non-small cell lung cancer with EGFR and/or ALK sensitive mutations) (Manegold C, et al, J Thorac Oncol. 2017 Feb; 12(2): 194-207.), renal cell carcinoma (Dudek AZ et al., J Clin Oncol. 2018; 36 (suppl; abstr 4558.), hepatocellular carcinoma (Stein S et al., J Clin Oncol, 2018, 36 (15_suppl): 4074.; bevacizumab in combination with atezolizumab was approved by the FDA in 2020 for the treatment of hepatocellular carcinoma), colorectal cancer (including MSI-H/dMMR and non-MSI-H/dMMR types) (Bendell JC et al. American Society of Clinical Oncology; May 29-June 2,2015; Chicago,IL.2015; abstract 704; Hochster HS et al. American Society of Clinical Oncology Gastrointestinal Cancers Symposium; January 19-21,2017; San Francisco,CA.2017; abstract 673), breast cancer (Yukinori Ozaki et al., AACR; Cancer Res 2020; 80(4Suppl):Abstract nr PD1-03.); the combination scheme of PD-1 antibody (pembrolizumab) and angiogenesis inhibitor (lenvatinib) has demonstrated good efficacy in the treatment of endometrial cancer, and was approved by the FDA for the treatment of endometrial cancer in 2019.

The results of a recently disclosed clinical trial showed: the PD-1/VEGF bispecific antibody ivonescimab (AK112) exhibited preliminary efficacy in solid tumors (the tumor type of most patients enrolled was previously considered to lack response to single-agent immunotherapy, such as serous ovarian cancer, MSS colorectal cancer, and other cancers) with an ORR of 25.5% and a disease control rate (DCR) of 63.8% ( J Immunother Cancer. 2024; 12(4): e008037.). The prior art also discloses: the efficacy of ivonescimab in combination with chemotherapy in the treatment of patients with locally advanced or metastatic nsq-NSCLC who have progressed after EGFR-TKI treatment is excellent.

The PD-1 antibody toripalimab has been approved for the treatment of melanoma, nasopharyngeal carcinoma, urothelial carcinoma, esophageal squamous cell carcinoma, and non-small cell lung cancer.

Studies in the prior art have also found: VEGF is closely related to tumor immunity (Immunity. 2013, 39(1): 61-73. Cancer Metastasis Rev, 2011, 30(1): 83-95.). In the tumor microenvironment, upregulated VEGF can act on endothelial cells and inhibit the expression of adhesion molecules, thereby inhibiting the adhesion and infiltration of T cells; and VEGF can also upregulate the expression of various immunosuppressive molecules such as PD-L1, PD-L1, TIM-3, IDO, etc., and inhibit the activation of effector T cells. Simultaneous inhibition of PD-1 signal and VEGF signal would have better anti-tumor effect, and animal experimental data and clinical data reported in the literature preliminarily support this strategy (Clin Exp Immunol, 2013, 172(3): 500-6. Sznol et al, ASCO, GU 2015). The combination of PD-1 monoclonal antibody and VEGFR2 monoclonal antibody, and the combination of PD-L1 monoclonal antibody and VEGF monoclonal antibody have shown good synergistic effects. The combination of bevacizumab (a monoclonal antibody targeting VEGF) with chemotherapy can significantly improve the survival rate of patients with colorectal cancer (Hurwitz et al. New Eng J Med 2004; 350: 2335-2342) or advanced non-squamous NSCLC (Sandler et al. J Clin Oncol 2005 23 (16s pt 1): 2ss).

Accordingly, the tenth aspect of the present application provides the use of the bispecific antibody according to the first aspect of the present application, or the conjugate according to the sixth aspect of the present application, or the pharmaceutical composition according to the ninth aspect of the present application in the preparation of a drug for preventing and/or treating a malignant tumor, or for at least inhibiting the development and deterioration of a malignant tumor. The malignant tumor is a PD-L1 protein-overexpressing (i.e. PD-L1 positive) and/or a VEGF protein-overexpressing (i.e. VEGF positive) tumor. For example, the malignant tumor is a solid tumor. At least, the bispecific antibodies of the present application can treat and/or inhibit the development and deterioration of lung cancer, colon cancer, colorectal cancer (CRC), melanoma or ovarian cancer. Preferably, the lung cancer is non-small cell lung cancer (NSCLC) or small cell lung cancer (SCLC). more preferably, the non-small cell lung cancer is squamous non-small cell lung cancer or non-squamous non-small cell lung cancer (nsq-NSCLC). more preferably, the non-squamous non-small cell lung cancer is locally advanced or metastatic nsq-NSCLC.

An eleventh aspect of the present application provides a method for treating or ameliorating a malignant tumor, comprising administering to a patient in need thereof the bispecific antibody according to the first aspect of the present application, or the conjugate according to the sixth aspect of the present application, or the pharmaceutical composition according to the ninth aspect of the present application; wherein the malignant tumor is a PD-L1 and/or VEGF overexpressing tumor; preferably, the malignant tumor is a solid tumor; more preferably, the malignant tumor is lung cancer, colon cancer, colorectal cancer (CRC), melanoma or ovarian cancer; more preferably, the lung cancer is non-small cell lung cancer (NSCLC) or small cell lung cancer (SCLC); more preferably, the non-small cell lung cancer is squamous non-small cell lung cancer or non-squamous non-small cell lung cancer (nsq-NSCLC); more preferably, the non-squamous non-small cell lung cancer is locally advanced or metastatic nsq-NSCLC.

A twelfth aspect of the present application provides a method of modulating an immune response and modulating tumor angiogenesis, comprising administering to a patient in need thereof the bispecific antibody according to the first aspect of the present application, or the conjugate according to the sixth aspect of the present application, or the pharmaceutical composition according to the ninth aspect of the present application; preferably, the modulating the immune response is achieved by blocking the binding of PD-1 to a receptor thereof, and the modulating tumor angiogenesis is achieved by blocking the binding of VEGF to a receptor thereof.

A thirteenth aspect of the present application provides the use of the bispecific antibody according to the first aspect of the present application, or the conjugate according to the sixth aspect of the present application, or the pharmaceutical composition according to the ninth aspect of the present application in the preparation of the following drug or reagent: a reagent for detecting the level of VEGF in a sample.

A fourteenth aspect of the present application provides the use of the bispecific antibody according to the first aspect of the present application, or the conjugate according to the sixth aspect of the present application, or the pharmaceutical composition according to the ninth aspect of the present application in the preparation of the following drug or reagent:

(1) a drug or reagent for detecting the level of VEGFA in a sample, or a drug or reagent for blocking the binding of VEGFA to VEGFR2, or a drug or reagent for down-regulating the activity or level of VEGFA, or a drug or reagent for abolishing the stimulating effect of VEGFA on the proliferation of vascular endothelial cells, or a drug or reagent for inhibiting the proliferation of vascular endothelial cells, or a drug or reagent for blocking tumor angiogenesis; and/or
(2) a drug or reagent for blocking the binding of PD-1 to PD-L1, or a drug or reagent for down-regulating the activity or level of PD-1, or a drug or reagent for abolishing the immunosuppression of PD-1 on the body, or a drug or reagent for promoting the secretion of IFN-$\gamma$ in T lymphocytes, or a drug or reagent for promoting the secretion of IL-2 in T lymphocytes.

In some embodiments of the present application, the use is for non-therapeutic purposes and/or non-diagnostic purposes.

A fifteenth aspect of the present application provides a method in vivo or in vitro, comprising the step of administering to a cell an effective amount of the bispecific antibody according to the first aspect of the present application, or the conjugate according to the sixth aspect of the present application, or the pharmaceutical composition according to the ninth aspect of the present application, wherein the method is selected from the group consisting of the following:

(1) a method for detecting the level of VEGFA in a sample, a method for blocking the binding of VEGFA to VEGFR2, a method for down-regulating the activity or level of VEGFA, a method for abolishing the stimulating effect of VEGFA on the proliferation of vascular endothelial cells, a method for inhibiting the proliferation of vascular endothelial cells, and a method for blocking tumor angiogenesis; and/or
(2) a method for blocking the binding of PD-1 to PD-L1, a method for down-regulating the activity or level of PD-1, a method for abolishing the immunosuppression of PD-1 on the body, a method for promoting the secretion of IFN-$\gamma$ in T

lymphocytes, and a method for promoting the secretion of IL-2 in T lymphocytes.

In some embodiments of the present application, the method in vitro is for non-therapeutic purposes and/or non-diagnostic purposes.

In the in vitro experiments of the present application, the anti-VEGF-anti-PD-1 bispecific antibodies can specifically bind to VEGF antigen, thereby inhibiting the proliferation of HUVEC cells; and the anti-VEGFA-anti-PD-1 bispecific antibody can promote the secretion of IFN-γ and/or IL-2 and activate the immune response.

A sixteenth aspect of the present application provides a method of preventing and/or treating a malignant tumor, comprising the step of administering to a subject an effective amount of the bispecific antibody according to the first aspect of the present application, or the conjugate according to the sixth aspect of the present application, or the pharmaceutical composition according to the ninth aspect of the present application.

A typical non-limiting range for a therapeutically or prophylactically effective amount of the bispecific antibody of the present application is 0.02-50 mg/kg. It should be noted that the dose may vary depending on the type and severity of the symptom requiring treatment. Further, one skilled in the art understands that for any particular patient, the particular dosing regimen should be adjusted over time according to the needs of the patient and the professional evaluation of the physician. The dose ranges given herein are for illustrative purposes only and do not limit the use or range of the pharmaceutical composition of the present application.

In the present application, the subject may be a mammal, preferably a primate, including a rhesus monkey, a cynomolgus monkey, a human, and the like.

A seventeenth aspect of the present application provides the bispecific antibody according to the first aspect of the present application, or the conjugate according to the sixth aspect of the present application, or the pharmaceutical composition according to the ninth aspect of the present application, for use in preventing and/or treating a malignant tumor.

An eighteenth aspect of the present application provides the bispecific antibody according to the first aspect of the present application, or the conjugate according to the sixth aspect of the present application, or the pharmaceutical composition according to the ninth aspect of the present application, for use in:

(1) detecting the level of VEGFA in a sample, or blocking the binding of VEGFA to VEGFR2, or down-regulating the activity or level of VEGFA, or abolishing the stimulating effect of VEGFA on the proliferation of vascular endothelial cells, or inhibiting the proliferation of vascular endothelial cells, or blocking tumor angiogenesis; and/or
(2) blocking the binding of PD-1 to PD-L1, or down-regulating the activity or level of PD-1, or abolishing the immunosuppression of PD-1 on the body, or promoting the secretion of IFN-γ in T lymphocytes, or promoting the secretion of IL-2 in T lymphocytes.

The present application is further described below with reference to specific examples. It should be understood that these examples are merely used to illustrate the present application and not to limit the scope of the present application. The reagents used in the experiment are commercially available unless otherwise specified. In addition, "parts" and "%" are based on mass, "room temperature" is 20-30°C, and "overnight" is 12-16 h unless otherwise specified.

**Example 1. Preparation of bispecific antibody molecules**

1. Construction of expression vector of bispecific antibody molecule

(1) Construction of expression vector of anti-PD-1 antibody

A first expression vector corresponding to the light chain of the anti-PD-1 antibody: a DNA sequence encoding 3E4L1 (the specific sequence is as set forth in SEQ ID NO: 21) was synthesized, digested by BSPQI and ligated to an HXT2 vector (appropriately modified based on pCDNA3.1 vector, the specific sequence is as set forth in SEQ ID NO: 22) to obtain an expression vector HXT2-3E4L1;

a second expression vector corresponding to the heavy chain of the anti-PD-1 antibody (i.e. JS001 HC): a DNA sequence encoding 1A6H2-QGQ (the specific sequence is as set forth in SEQ ID NO: 23) was synthesized, digested by BSPQI and ligated to an HXT4S vector (appropriately modified based on pCDNA3.1 vector, the specific sequence is as set forth in SEQ ID NO: 24) to obtain an expression vector HXT4S-1A6H2-QGQ.

(2) Construction of expression vector of anti-VEGF single-domain antibody

A DNA sequence encoding IgG4-FC (the specific sequence is as set forth in SEQ ID NO: 25) was synthesized, digested by BSPQI-NotI and ligated to an HXT4S vector to obtain an expression vector HX4-FC;

a DNA sequence encoding 16C2-1 (the specific sequence is as set forth in SEQ ID NO: 26) was synthesized and inserted into an HX4-FC vector (the specific sequence is as set forth in SEQ ID NO: 27) through the SapI-SapI site to obtain an expression vector HX4-16C2-1Fc.

(3) Construction of expression vector of bispecific antibody molecule

1) Bispecific antibody molecule 23:

the method for constructing the first expression vector HXT2-3E4L1 was the same as the method for constructing the first expression vector in "(1) Construction of expression vector of anti-PD-1 antibody";
a DNA sequence encoding 16C2-1-JS001 HC (the specific sequence is as set forth in SEQ ID NO: 28) was synthesized, digested by EcoRI-NheI and ligated to an HXT4S vector to obtain a second expression vector HXT4S-16C2-1-JS001 HC.

2) Bispecific antibody molecule 24:

the method for constructing the first expression vector HXT2-3E4L1 was the same as the method for constructing the first expression vector in "(1) Construction of expression vector of anti-PD-1 antibody";
a DNA sequence encoding IgG4-16C2-1 (the specific sequence is as set forth in SEQ ID NO: 29) was synthesized, digested by NheI-NotI and ligated to the HXT4S-1A6H2-QGQ vector (that is,
the second expression vector corresponding to the heavy chain of the anti-PD-1 antibody) to obtain a second expression vector HXT4s-JS001 HC-16C2-1.

3) Bispecific antibody molecule 27:

the method for constructing the first expression vector HXT2-3E4L1 was the same as the method for constructing the first expression vector in "(1) Construction of expression vector of anti-PD-1 antibody";
a DNA sequence encoding JS001 HC-CH1-16C2-1 (the specific sequence is as set forth in SEQ ID NO: 30) was synthesized, digested by SapI-SapI and ligated to the HX4-FC vector to obtain a second expression vector HXT4S-JS001 HC-CH1-16C2-1-FC.

4) Bispecific antibody molecule 28:

A DNA sequence encoding 16C2-1-3E4L1 (the specific sequence is as set forth in SEQ ID NO: 34) was synthesized, digested by SapI-SapI and ligated to an HXT2 vector to obtain a first expression vector HXT2-16C2-1-3E4L1;
the method of constructing the second expression vector HXT4S-1A6H2-QGQ was the same as the method of constructing the second expression vector in "1. a second expression vector corresponding to the heavy chain of the anti-PD-1 antibody".

2. Expression of bispecific antibody molecule

The CHO-K1 cells being cultured were counted, and passaging for expansion was performed with CD CHO (Thermofisher, 12490-003) medium when the cell density was $2 \times 10^6$/mL - $6 \times 10^6$/mL. One day prior to transfection, the cell density was diluted to $1.5 \times 10^6$/mL - $2.0 \times 10^6$/mL. On the next day, transfection was performed when the cell density reaches about $3.0 \times 10^6$/mL - $4.0 \times 10^6$/mL. CD CHO medium with a volume of one tenth of the transfection volume was added first, followed by the addition of plasmids (prepared in "1. Construction of expression vector of bispecific antibody molecule" above) at 1-2 $\mu$g per mL of transfection volume, and finally PEI (polyethyleneimine, Polysciences, 24765-1) at 3-14 $\mu$g/mL was added. After mixed evenly, the mixture was incubated at room temperature for no more than 30 min. Finally the transfection mixture was slowly added to the treated CHO-K1 cells above with a cell density of about $3.0 \times 10^6$/mL - $4.0 \times 10^6$/mL, with mixing evenly during the addition. The transfected mixture was placed in a carbon dioxide shaker for culture under the culture conditions of 36.5°C, 110 rpm, 7% (volume fraction) $CO_2$. On the first day after transfection, 4 vol% Cell Boost 7a (Hyclone, SH31026.05) and 0.4 vol% Cell Boost 7b (Hyclone, SH31027.04CN) was supplemented, and then supplemented every two days and samples were collected 5-9 days after transfection.

3. Purification of bispecific antibody molecule

After the culture was completed, the pellet was discarded after centrifugation at 1000 g for 5 min, then centrifugation was performed at 12000 g for 30 min, and the cell supernatant was collected and sterile-filtered through a 0.22 μm filter cup. The obtained system was purified by AKTA Avant purifier. First, the LX column (Cytiva, 17547405) loaded with mabselect sure was subjected to CIP with 0.1 M NaOH for 15-20 min, then equilibrated with PBS buffer until each curve leveled off, and then the sample was loaded. After the sample loading was completed, the column was respectively rinsed with affinity chromatography elution buffer, and finally the protein of interest was eluted with acetic acid-sodium acetate buffer at pH 3.6. The sample was neutralized with 1 M Tris buffer to adjust the pH = 5.5. A part of the sample was sent to SEC-HPLC (molecular exclusion high performance liquid chromatography) for detecting the purity of the sample. The remaining samples were purified in the next step; Eshmuno CPX (Merck, 1200830100) was selected for purification. The equilibration solution was 50 mM acetic acid-sodium acetate system at pH 5.5, and the eluent was 50 mM acetic acid-sodium acetate + 1 M NaCl buffer system at pH 5.5. The linear elution method was used to collect the protein of interest, and the final SEC-HPLC monomer purity reached more than 95%.

The schematic structural diagram of the prepared bispecific antibody molecule antibody 23 is shown in FIG. 2a, the schematic structural diagram of the antibody 24 is shown in FIG. 2b, the schematic structural diagram of the antibody 27 is shown in FIG. 2c, and the schematic structural diagram of the antibody 28 is shown in FIG. 2d. The amino acid sequences comprised in the bispecific antibody molecules are shown in Table 1 below. The DotAb in FIGs. 2a to 2d is a single-domain antibody (VHH) that specifically binds to VEGF (anti-VEGF) and is a VHH of the sequence as set forth in SEQ ID NO: 12.

Table 1 Amino acid sequences comprised in the bispecific antibody molecules

|  | Antibody 23 | Antibody 24 | Antibody 27 | Antibody 28 |
|---|---|---|---|---|
| The first polypeptide chain | SEQ ID NO: 13 | SEQ ID NO: 13 | SEQ ID NO: 13 | SEQ ID NO: 17 |
| The second polypeptide chain | SEQ ID NO: 14 | SEQ ID NO: 15 | SEQ ID NO: 16 | SEQ ID NO: 18 |

**Example 2. Stability of bispecific antibody**

1. Thermostability of bispecific antibody

DSF (differential scanning fluorescence technique) was used to investigate the stability of bispecific antibody in buffer system (20 mM histidine-histidine hydrochloride buffer, 230 mM sucrose, prepared in ultrapure water, pH 5.0).

The bispecific antibody sample was exchanged into the above buffer system, and the sample concentration was controlled at about 10 mg/mL, and the detection was performed by nanoDSF (microdifferential scanning fluorescence technology). The results are shown in Table 2. The thermal conversion temperature (Tm) of the bispecific antibody 23, antibody 24 and antibody 27 provided in the present application is above 56°C, showing good thermal stability.

Table 2 Thermostability of bispecific antibody

| Sample | Tm1 (°C) | Tm2 (°C) | Tm3 (°C) |
|---|---|---|---|
| Antibody 23 | 59.1 | / | / |
| Antibody 24 | 63.8 | / | / |
| Antibody 27 | 56.2 | 66.5 | 76.6 |
| Notes: "/" denotes absence. | | | |

2. Long-term stability of bispecific antibody

The bispecific antibody sample was exchanged into a pH 5.0 buffer system (20 mM histidine-histidine hydrochloride buffer, 230 mM sucrose), and the concentration of the bispecific antibody sample was controlled to about 10 mg/mL. The sample was subpackaged into vials according to 500 μL/vial, and placed in a medical refrigerator at 5°C, and the stability was investigated at 0, 2 and 4 weeks. Stability was assessed by the following parameters: (a) measuring the content of antibody monomers, polymers and fragments by SEC-HPLC (molecular exclusion high performance liquid chromatography); (b) detecting the molecular weight of the antibody by CE-SDS (capillary electrophoresis-sodium dodecyl sulfate) method; (c) detecting the isoelectric point of each component sample by capillary electrophoresis instrument (Maurice instrument) equipped with icIEF cartridge; after the test article was diluted with iCIEF mixed solution, the capillary electrophoresis instrument (Maurice) was used for detection; components with different isoelectric points were focused at different positions to achieve the effect of focusing and separation; (d) detected the biological activity of the antibody by ELISA method and reporter gene method.

The (a) measuring the content of antibody monomers, polymers and fragments by SEC-HPLC (molecular exclusion high performance liquid chromatography) comprises the following steps: HPLC (Waters e2695 instrument) equipped with SEC column (Waters XBridge BEH, 7.8 mm × 30 cm, 3.5 μm, 200 Å) was used to detect SEC purity. Each sample was respectively diluted to 4.0 mg/mL with mobile phase, the mixture was mixed evenly and then added to the lined tube, and directly injected for analysis; the mobile phase consisted of 50 mM phosphate solution (containing 300 mM sodium chloride, 30 mM disodium hydrogen phosphate dodecahydrate, and 20 mM sodium dihydrogen phosphate dihydrate, prepared in ultrapure water), pH 6.8 ± 0.2. The area normalization method was used to calculate the relative percentages of major peaks, polymers and fragments. The pretreatment method and test method of the reference article were the same as those of the sample. The test parameters are shown in Table 3 below:

Table 3 SEC-HPLC purity test parameters

| Chromatographic conditions | Chromatographic parameters |
|---|---|
| Chromatographic column | Waters XBridge BEH, 7.8 mm × 30 cm, 3.5 μm, 200 Å |
| Detection wavelength | 280 nm |
| Column oven | 25 ± 3°C |
| Sample pan temperature | 5 ± 3°C |
| Flow rate | 0.5 mL/min |
| Injection volume | 25 μL (sample concentration of 4.0 mg/mL) |
| Mobile phase | 50 mM phosphate, 300 mM sodium chloride, pH 6.8 ± 0.2 |
| Elution mode | Isocratic elution |
| Elution time | 30 min |

The (b) detecting the molecular weight of the antibody by R-CE-SDS (reduced capillary electrophoresis-sodium dodecyl sulfate method) comprises the following steps: the rCE-SDS purity test was performed using a capillary electrophoresis instrument (Maurice instrument). Each sample was diluted to 1 mg/mL with a diluent (1 wt% SDS + 40 mM phosphate buffer, pH 6.5), then 95 μL of the diluted sample and 5 μL of 2-mercaptoethanol were mixed evenly in a sample tube, and the mixture was centrifuged at 3000 rpm for 30 s at room temperature, and then incubated at 70°C for 15 min. After the incubation was completed, the obtained system was cooled to room temperature and centrifuged at 12000 rpm at room temperature for 5 min. 75 μL of sample solution was taken from each sample tube and added to a 96-well plate while avoiding bubbles, and the plate was centrifuged at 1000 g at room temperature for 10 min, and the capillary electrophoresis instrument (Maurice) was used for detection. The pretreatment method and test method of the reference article were the same as those of the sample.

Detecting the molecular weight of the antibody by NR-CE-SDS (non-reduced capillary electrophoresis-sodium dodecyl sulfate method) comprises the following steps: the nrCE-SDS purity test was performed using a capillary electrophoresis instrument (Maurice instrument). The amount of the diluent and the sample to be added when diluting each sample to 1 mg/mL was calculated according to the final volume of 100 μL, and then the diluent (1 wt% SDS + 40 mM phosphate buffer, pH 6.5), 5.0 μL of 0.25 M NEM (N-ethylmaleimide) and the sample were added to a 1.5 mL sample tube in sequence, and the mixture was mixed evenly. The obtained system was centrifuged at 3000 rpm at room temperature for 30 s, and then incubated at 70°C for 5 min. After the incubation was completed, the obtained system was cooled to room temperature and centrifuged at 12000 rpm at room temperature for 5 min. 75 μL of sample solution was taken from each sample tube and added to a 96-well plate while avoiding bubbles, and the plate was centrifuged at 1000 g at room temperature for 10 min, and the capillary electrophoresis instrument (Maurice instrument) was used for detection. The pretreatment method and test method of the reference article were the same as those of the sample.

(c) detecting the isoelectric point of each component sample by capillary electrophoresis instrument (Maurice instrument) equipped with icIEF cartridge, components with different isoelectric points were focused at different positions to achieve the effect of focusing and separation. The step of detecting the isoelectric point comprises: The sample (reference article/test article) was diluted to 1.0 mg/mL with ultrapure water. 20 μL of sample solution (1.0 mg/mL) or ultrapure water (blank sample) was taken and mixed evenly respectively with 80.0 μL of icIEF Master Mix in sample tubes, and the sample tubes were centrifuged at 10000 rpm for 5 min. 75.0 μL of the supernatant was taken from the sample tube and added to the 96-well plate, taking care to avoid bubbles, and then the 96-well plate was centrifuged in a centrifuge at 1000 g for 5 min to remove bubbles. Detection was performed using a capillary electrophoresis instrument (Maurice instrument). The pretreatment method and test method of the reference article were the same as those of the sample. Components with different isoelectric points were focused at different positions to achieve the effect of focusing and separation. The mixture solution icIEF Master Mix was prepared as shown in Table 4 below and the analytical method parameters are shown in Table 5.

Table 4 Table of icIEF Master Mix preparation

| Reagent name | Volume required per sample (μL) | Total volume required (μL) |
|---|---|---|
| Methylcellulose (1 w/v% MC) | 35.0 | |
| Pharmalyte 3-10 | 4.0 | |
| pI Marker 5.12 | 0.1 | (Number of blank samples + number of reference articles + number of samples +1) × reagent volume required per sample |
| pI Marker 8.40 | 0.5 | |
| 8 M Urea | 25.0 | |
| Ultrapure water | 15.4 | |

Table 5 Parameters of the sample run method

| Method | Value |
|---|---|
| Separation | 1.0 min, 1500 V; 8.0 min, 3000 V |
| Detection | 5 Exposures |
| SampleLoad | 55 s |
| pI Marker | pI Marker 5.12 620 pixels; pI Marker 8.40 1550 pixels |
| Ampholytes | Pharmalyte 3-10 |
| Additives | Urea |

(d) detecting the biological activity of the antibody by ELISA method comprises the following steps: an indirect method was used, and Human PD-1 and Human VEGF were used as antigens to coat on a 96-well plate. The antibody could bind to Human PD-1 and Human VEGF. Mouse Anti-Human IgG4 Fc-HRP (HRP-labeled mouse anti-human IgG4 (Fc) secondary antibody, manufacturer: Southern Biotech; catalog number: 9200-05) could specifically bind to antibodies bound to solid phase antigens (Human PD-1 and Human VEGF), and could catalyze TMB (trimethyl borate) under the action of hydrogen peroxide, resulting in the development of a blue color. The shade of blue color was positively correlated with the amount of Mouse Anti-Human IgG4 Fc-HRP binding. The reaction was terminated by the addition of 2 M hydrochloric acid, turning the solution yellow. The absorbance (OD value) was measured with a microplate reader at a wavelength of 450 nm/620 nm, and the effective binding activity of the standard curve ($EC_{50}$) was used to investigate the ability of the bispecific antibody to bind to Human PD-1 and Human VEGF, respectively. Details are shown in Tables 6 and 7 below:

Table 6 ELISA experimental method of antibody binding to PD-1

| Experimental procedure | Sample name | Concentration | Temperature | Time |
|---|---|---|---|---|
| Coating | Human PD-1 | 0.3 μg/mL | | 90 min |
| Blocking | BSA (Bovine Serum Albumin) | 2% (1 g of BSA plus 50 mL of PBS) | 37°C | 90 min |
| Sample | Antibody test article | starting at 1.0 μg/mL, 2.5-fold gradient dilution | | 60 min |
| Detection | Mouse Anti-Human IgG4 Fc-HRP | 5000-fold dilution | | 60 min |

(continued)

| Experimental procedure | Sample name | Concentration | Temperature | Time |
|---|---|---|---|---|
| Coloration | Chromogenic solution | 0.1 mg/mL | | 15 min |

Table 7 ELISA experimental method of antibody binding to VEGF

| Experimental procedure | Sample name | Concentration | Temperature | Time |
|---|---|---|---|---|
| Coating | Human/Cynomolgus VEGF/-VEGFA/VE GF165 Protein | 0.5 μg/mL | 37°C | 90 min |
| Blocking | BSA | 2% | | 90 min |
| Sample | Antibody test article | starting at 1.0 μg/mL, 3-fold gradient dilution | | 60 min |
| Detection | Mouse Anti-Human IgG4 Fc-HRP | 5000-fold dilution | | 60 min |
| Coloration | Chromogenic solution | 0.1 mg/mL | | 15 min |

Detecting the biological activity of the antibody by reporter gene method comprises the following steps:

(1) VEGF end: vascular endothelial growth factor (VEGF), also known as vascular permeability factor (VPF), is a highly specific pro-vascular endothelial growth factor, which has the effects of promoting the increase of vascular permeability, the degeneration of extracellular matrix, the migration and proliferation of vascular endothelial cells and angiogenesis. The high-affinity receptor that specifically binds to VEGF vascular endothelial growth factor is referred to as vascular endothelial growth factor receptor (VEGFR), which is mainly divided into 3 types: VEGFR-1, VEGFR-2, and VEGFR-3. VEGFR-1 and VEGFR-2 are mainly distributed on the surface of tumor vascular endothelium and regulate tumor angiogenesis; VEGFR-3 is mainly distributed on the surface of lymphoid endothelium and regulates tumor lymphangiogenesis. Target cells 293 VEGFR2 overexpressing VEGFR2 and luciferase reporter gene system, and recombinant VEGF (R&D systems, cat# 11066-HNAB) protein were used to detect the biological activity of the antibody in cell in the present application. Target cell 293 VEGFR2 cells were seeded into a 96-well flat-bottom white plate (corning, cat# 3917) at a number of 50,000 cells per well, and the gradiently diluted antibody to be tested was added at 25 μL/well (final concentration of 10.2 pM-66.7 nM); a diluted VEGF protein at working concentration of 25 ng/mL was then added and co-incubated at 37°C for a total of 5-6 h. Finally, luciferase substrate One-Lite (Vazyme, cat# DD1203-03-AB) was added at 40 μL/well and chemiluminescence was detected.
(2) PD-1 end: NFAT is a key transcription factor for downstream signaling of T cell receptor activation and is essential for the secretion of IL-2 as well as proliferation response of T cell. The antibody can specifically bind to PD-1 on the cell surface and block PD-1/PD-L1 interaction, thereby activating the downstream NFAT signaling pathway. Two cell lines are included in the system of the present application: Jurkat PD-1 effector cells (purchased from Promega), a cell line that stably expresses human PD-1 and a luciferase reporter gene conjugated to NFAT on Jurkat cells; a CHO PDL1 target cell line (purchased from Promega), a cell line that stably expresses CD3 scFv and human PDL1 on CHO-K1 cells. When PD-1 on the surface of effector cells binds to PDL1 on the surface of target cells, the NFAT signaling pathway is inhibited, and the expression of the luciferase reporter gene conjugated to NFAT is inhibited. The antibody can specifically bind to PD-1 and block the PD-1/PDL1 interaction, thereby activating the downstream NFAT signaling pathway. The detection protocol of the present application is as follows: on the first day, target cells, i.e., PD-L1 aAPC/CHO-K1 cells were plated at $5 \times 10^4$ cells/well and cultured overnight in a cell incubator; the next day, the experimental antibody (final concentration was 7.6 pM-500 nM) was prepared and added to the cell plate at 40 μL/well, and then effector cells, i.e., PD-1 Effector cells were added at $1 \times 10^5$ cells/well; and the obtained system was cultured in a 37°C, 5% (volume fraction) $CO_2$ incubator for 4-6 h; luciferase substrate was added at 40 μL/well, the plate was read by a multifunctional microplate reader and the data was analyzed.

The detection results showed that the purity (SEC-HPLC method, R-CE-SDS (reduction electrophoresis method), NR-CE-SDS (non-reduction electrophoresis method) and icIEF) and biological activity of the 3 bispecific antibody molecules did not change significantly, and they had good long-term stability. The specific results are shown in Table 8.

Table 8 Stability of bispecific antibody stored at 5°C for 4 weeks

| Sample | Time | SEC-HPLC (%) | | | icIEF(%) | | | NR-CE-SDS (%) | R-CE-SDS (%) | Binding activity (%) | | Cell activity (%) | |
|---|---|---|---|---|---|---|---|---|---|---|---|---|---|
| | | Polymer | Monomer | Fragment | group1 | group2 | group3 | | | PD -1 end | VEG F end | PD -1 end | VEG F end |
| Antibody 23 | 0 week | 1.1 | 98.9 | ND | 35.7 | 52.9 | 11.4 | 99.8 | 99.8 | 105 | 99 | / | 108 |
| | 2 week | 1.3 | 98.7 | ND | 38.3 | 52.3 | 9.3 | 99.8 | 1000 | / | 104 | / | / |
| | 4 week | 1.4 | 98.6 | ND | 39.5 | 52.2 | 8.3 | 99.7 | 98.8 | 97 | 81 | / | 92 |
| Antibody 24 | 0 week | 1.7 | 98.3 | ND | 33.5 | 57.7 | 8.7 | 98.7 | 98.9 | 94 | 100 | / | 94 |
| | 2 week | 2.2 | 97.8 | ND | 36.3 | 54.0 | 9.6 | 99.7 | 99.2 | / | 85 | / | / |
| | 4 week | 1.8 | 98.2 | ND | 34.1 | 51.3 | 14.6 | 99.5 | 97.4 | 86 | 102 | / | 100 |
| Antibody 27 | 0 week | 0.8 | 98.5 | 0.8 | 30.2 | 60.4 | 9.4 | 99.0 | 99.3 | 95 | 97 | 102 | 89 |
| | 2 week | 0.7 | 98.3 | 1.0 | 30.9 | 60.2 | 8.9 | 99.0 | 99.4 | 96 | 92 | 114 | 93 |
| | 4 week | 0.7 | 98.0 | 1.3 | 30.3 | 60.9 | 8.8 | 98.9 | 99.3 | 107 | 104 | 104 | 111 |

Notes: "/" denotes not determined, and "ND" denotes not detected.

3. Serum stability of bispecific antibody

The bispecific antibody sample was exchanged into a pH 5.0 buffer system (20 mM histidine-histidine hydrochloride buffer, 230 mM sucrose), and the concentration of the bispecific antibody sample was controlled to about 10 mg/mL. Then the sample was diluted to 1 mg/mL with human serum, subpackaged into vials according to 500 μL/vial, and the vials were placed in an incubator at 37°C, and the stability was investigated at 0, 2 and 4 weeks. The biological activity of the antibody was detected by ELISA method and reporter gene method. The specific detection method was the same as the method corresponding to "2. Long-term stability of bispecific antibody" above.

The detection results showed that after 4 weeks of storage at 37°C, the biological activities of the serum stability samples of antibody 23, antibody 24 and antibody 27 did not change significantly, and these antibodies had good serum stability. The specific results are shown in Table 9.

Table 9 Stability of serum stability sample of bispecific antibody stored at 37°C for 4 weeks

| Sample | Time | Binding activity (%) | | Cell activity (%) | |
|---|---|---|---|---|---|
| | | PD-1 end | VEGF end | PD-1 end | VEGF end |
| Antibody 23 | 0 week | 121 | 98 | / | 107 |
| | 2 week | 95 | 96 | / | 80 |
| | 4 week | 82 | 79 | / | 70 |
| Antibody 24 | 0 week | 106 | 104 | / | 105 |
| | 2 week | 63 | 81 | / | 86 |
| | 4 week | 89 | 95 | / | 87 |
| Antibody 27 | 0 week | 93 | 101 | 77 | 88 |
| | 2 week | 86 | 86 | 73 | 79 |
| | 4 week | 83 | 78 | 71 | 68 |
| Notes: "/" denotes no determination. | | | | | |

## Example 3. Determination of kinetic parameters of bispecific antibody

1. Determination of kinetic parameters of bispecific antibody and antigen PD-1

The binding of 5 μg/mL JS001 (recombinant humanized anti-PD-1 monoclonal antibody reference article, prepared by Suzhou Junmeng Biopharm Co., Ltd. according to the antibody sequence of the common name Toripalimab, wherein the amino acid sequence of LC is as set forth in SEQ ID NO: 31 and the amino acid sequence of HC is as set forth in SEQ ID NO: 32), antibody 23, antibody 24, antibody 27 and antibody 28 to 210 nM and 70 nM recombinant human PD-1 (prepared by Suzhou Junmeng Biopharm Co., Ltd. according to the sequence of NCBI GenBank: NM_005018) was respectively captured using a molecular interaction analyzer Octet RED96e (Sartorius) to detect the kinetic signals of association and dissociation. The association and dissociation curves were fitted using the analytical software Octet Data Analysis 12.0 and the affinity $K_D$ Value was calculated.

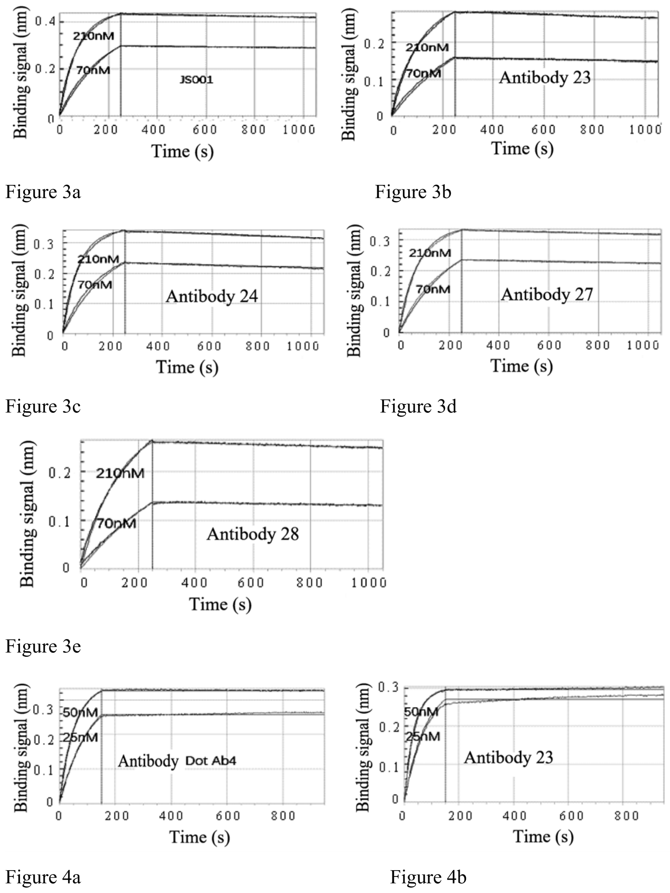

The specific results are shown in FIG. 3a-FIG. 3e and Table 10, wherein FIG. 3a is a graph showing the association and dissociation curves of antibody JS001 to human PD-1; FIG. 3b is a graph showing the association and dissociation curves of antibody 23 to human PD-1; FIG. 3c is a graph showing the association and dissociation curves of antibody 24 to human PD-1; FIG. 3d is a graph showing the association and dissociation curves of antibody 27 to human PD-1; FIG. 3e is a graph showing the association and dissociation curves of antibody 28 to human PD-1. As can be seen from FIG. 3a-FIG. 3e and Table 10: antibody JS001, antibody 23, antibody 24, antibody 27 and antibody 28 all had binding activity to human PD-1 with similar affinity; the above results indicated that the bispecific antibody 23, antibody 24, antibody 27, and antibody 28 all maintained the affinity of their original anti-PD-1 monoclonal antibody JS001.

Table 10 Data on the association and dissociation of JS001, antibody 23, antibody 24, antibody 27 and antibody 28 molecules to human PD-1

| Antigen name | Antibody name | $k_a$ (1/Ms) | $k_d$ (1/s) | $K_D$ (M) |
|---|---|---|---|---|
| | JS001 | 6.73E+04 | 3.93E-05 | 5.84E-10 |

(continued)

| Antigen name | Antibody name | $k_a$ (1/Ms) | $k_d$ (1/s) | $K_D$ (M) |
|---|---|---|---|---|
| Recombinant human PD-1 | Antibody 23 | 4.48E+04 | 7.73E-05 | 1.73E-09 |
| | Antibody 24 | 7.76E+04 | 9.38E-05 | 1.21E-09 |
| | Antibody 27 | 6.44E+04 | 5.57E-05 | 8.64E-10 |
| | Antibody 28 | 3.58E+04 | 5.96E-05 | 1.67E-09 |

2. Determination of kinetic parameters of bispecific antibody and VEGFA

The binding of 5 μg/mL DotAb4 (the DotAb4 sequence was obtained from DotBio and expressed, and the amino acid sequence of DotAb4 is as set forth in SEQ ID NO: 33, i.e. **EVQLVESGGGLVQPGGSLRLSCAISGFSLAATDIDW VRQAPGKGLEWVARIFSPSDF TDYADSVKGRFTISADTSKNTVYLQMNSLRAEDTAVYYCGRPDTYAYNIFLDTPTY N ALHYRGQGTLVTVSS**GGGGSESKYGPPCPPCPAPEFLGGPSVFLFPPKPKDTLMISRTPE VTCVVVDVSQEDPEV QFNWYVDGVEVHNAKTKPREEQFNSTYRVVSVLTVLHQDWLN GKEYKCKVSNKGLPSSIEKTISKAKGQPREPQVY TLPPSQEEMTKNQVSLTCLVKGFYPS DIAVEWESNGQPENNYKTTPPVLDSDGSFFLYSRLTVDKSRWQEGNVFSC SVMHEALHN HYTQKSLSLSLSLGK, wherein the bold and underlined parts are the VHH sequence targeting VEGF), antibody 23, antibody 24, and antibody 27 to 50 nM and 25 nM recombinant human VEGFA (purchased from Sino Biological, catalog number 11066-HNAB) was respectively captured using a molecular interaction analyzer Octet RED96e to detect the kinetic signals of association and dissociation. The binding of 5 μg/mL antibody 28 to 25 nM, 12.5 nM VEGFA was captured using a molecular interaction analyzer Octet to detect the kinetic signals of association and dissociation. The association and dissociation curves were fitted using the analytical software Octet Data Analysis 12.0 and the affinity $K_D$ Value was calculated.

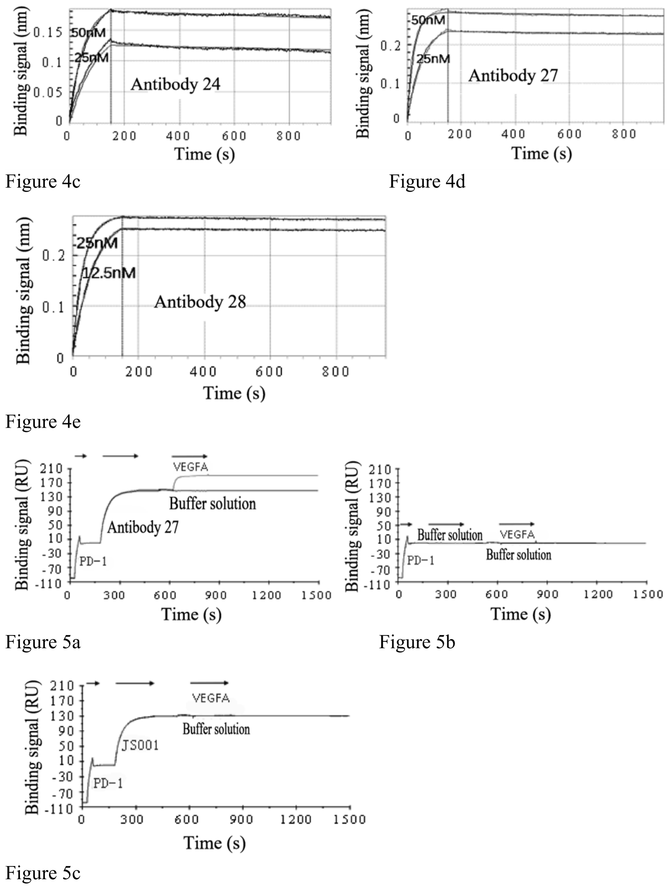

The specific results are shown in FIG. 4a-FIG. 4e and Table 11, wherein FIG. 4a is a graph showing the association and dissociation curves of antibody DotAb4 to human VEGFA; FIG. 4b is a graph showing the association and dissociation curves of antibody 23 to human VEGFA; FIG. 4c is a graph showing the association and dissociation curves of antibody 24 to human VEGFA; FIG. 4d is a graph showing the association and dissociation curves of antibody 27 to human VEGFA; FIG. 4e is a graph showing the association and dissociation curves of antibody 28 to human VEGFA. As can be seen from FIG. 4a-FIG. 4e and Table 11: antibody DotAb4, antibody 23, antibody 24, antibody 27, and antibody 28 all have strong binding activity to human VEGFA, with $K_D$ all less than 0.5 nM; the above results indicated that the bispecific antibody 23, antibody 24, antibody 27, and antibody 28 all maintained the affinity of their original anti-VEGFA nanobody DotAb4.

Table 11 Data on the association and dissociation of DotAb4, antibody 23, antibody 24, antibody 27 and antibody 28 to human VEGFA

| Antigen name | Antibody name | $k_a$ (1/Ms) | $k_d$ (1/s) | $K_D$ (M) |
|---|---|---|---|---|
| Recombinant human VEGFA | Antibody DotAb4 | 3.75E+05 | <1.00E-05 | <2.66E-11 |
| | Antibody 23 | 5.38E+05 | <1.00E-05 | <1.86E-11 |
| | Antibody 24 | 4.14E+05 | 7.92E-05 | 1.91E-10 |
| | Antibody 27 | 8.15E+05 | 4.24E-05 | 5.21E-11 |
| | Antibody 28 | 1.28E+06 | 1.88E-05 | 1.47E-11 |

3. Determination of the binding activity of bispecific antibody to PD-1 and VEGFA simultaneously

The ability of bispecific antibody 27 to bind simultaneously to two target antigens, human PD-1 and human VEGFA, was tested using a molecular interaction analyzer Biacore T200 (GE Healthcare Life Sciences). The testing process included: 20 μg/mL anti-mouse Fc fragment antibody was conjugated to capture PD-1 antigen with murine Fc tags. For antibody 27 in the sample group to be tested, that is, for the detection of the ability of antibody 27 to bind PD-1 and VEGFA simultaneously, 2 μg/mL PD-1 was first captured, then 100 nM antibody 27 was injected, and finally 80 nM human VEGFA or Biacore system buffer (HBS-EP +) (10 mM buffer salt HEPES, 150 mM sodium chloride, 3 mM ethylenediamine tetraacetic acid, 0.05 vol% detergent Surfactant P20) was injected to detect the binding signal produced by human VEGFA, which was the signal indicating that antibody 27 bound to PD-1 and then to VEGFA. At the same time, a negative control group was set up, 2 μg/mL PD-1 was first captured, then buffer was injected, and finally 80 nM human VEGFA or buffer was injected to detect the binding signal produced by human VEGFA, which was used to determine whether VEGFA would produce non-specific binding to PD-1. At the same time, another negative control group was set up. 2 μg/mL PD-1 was first captured, then 100 nM anti-PD-1 monoclonal antibody JS001 was injected, and finally 80 nM human VEGFA or buffer was injected to detect the binding signal produced by human VEGFA, which was used to determine whether VEGFA produced a non-specific binding signal to the anti-PD-1 antibody region.

The specific results are shown in FIG. 5a-FIG. 5c and Table 12, wherein FIG. 5a is a graph showing the results of binding of the antibody 27 in the sample group to be tested to two antigens, i.e., human PD-1 and human VEGFA, simultaneously; FIG. 5b is a graph showing the results of non-specific binding of human VEGFA to human PD-1 in the negative control group; FIG. 5c is a graph showing the results of non-specific binding of human VEGFA to JS001 in the negative control group. As can be seen from FIG. 5a-FIG. 5c and Table 12: for the sample group to be tested, the binding signal produced by VEGFA was 41.2 RU, indicating that bispecific antibody 27 could bind to two antigens, i.e., human PD-1 and human VEGFA, simultaneously; the signal produced by VEGFA in the two negative control groups was only 1.0 RU or 1.5 RU, indicating that VEGFA does not non-specifically bind to the antigen PD-1, nor does it non-specifically bind to the Fab or Fc region of the anti-PD-1 antibody JS001.

Table 12 Data on simultaneous binding of antibody 27 to human PD-1 and human VEGFA

| Group | Captured antigen | Capture signal (RU) | Bound antibody | Bound antibody signal (RU) | Bound antigen | Bound antigen signal (RU) |
|---|---|---|---|---|---|---|
| Sample group to be tested | Human PD-1 | 99 | Antibody 27 | 145 | Human VEGFA | 41.2 |
| Negative control group | Human PD-1 | 98 | Buffer solution | -0.7 | Human VEGFA | 1.0 |
| Negative control group | Human PD-1 | 98 | JS001 | 128 | Human VEGFA | 1.5 |

**Example 4. Detection of binding activity of bispecific antibody to antigen by ELISA method**

1. Detection of binding of bispecific antibody to antigen PD-1 by ELISA method

Human PD-1 was diluted to 0.3 μg/mL with PBS (manufacturer: Hyclone, catalog number: SH30256.01), and the mixture was added to the ELISA plate at 100 μL/well, and the plate was placed in a 37°C incubator and coated for 90 min; the plate was washed; 2% BSA (bovine serum albumin, 2% means 1 g BSA plus 50 mL PBS) was added to the plate at 200 μL/well, and the plate was placed in a 37°C constant temperature incubator for incubation for 90 min; the plate was washed; the samples were diluted to 1000 ng/mL with 2% BSA, followed by dilution to 0.0419 ng/mL in a 2.5-fold gradient. All samples were added to the ELISA plate at 100 μL/well, and the plate was placed in a 37°C constant temperature incubator for incubation for 60 min; the plate was washed; Horseradish peroxidase (HRP)-conjugated murine anti-human antibody IgG4 (manufacturer: Southern Biotech, catalog number: 9200-05) (Fc-specific) was diluted 5000-fold with 2% BSA, and the mixture was added to the ELISA plate at 100 μL/well, and the plate was placed in a 37°C constant temperature incubator for incubation for 60 min; the plate was washed; the chromogenic solution TMB (trimethyl borate, manufacturer: Sigma, catalog number: T2885) at 0.1 mg/mL was added at 100 μL/well, while avoiding bubbles, and coloration was performed at 37°C protected from light for 15 min; 2 M hydrochloric acid solution was finally added at 100 μL/well to terminate the reaction, while avoiding bubbles, the reading of microplate reader was completed within 10 min (detection wavelength: 450 nm; reference wavelength: 620 nm), and the $EC_{50}$ (concentration for 50% of maximum effect) was obtained by fitting using the four-parameter logarithmic regression (4PL) model.

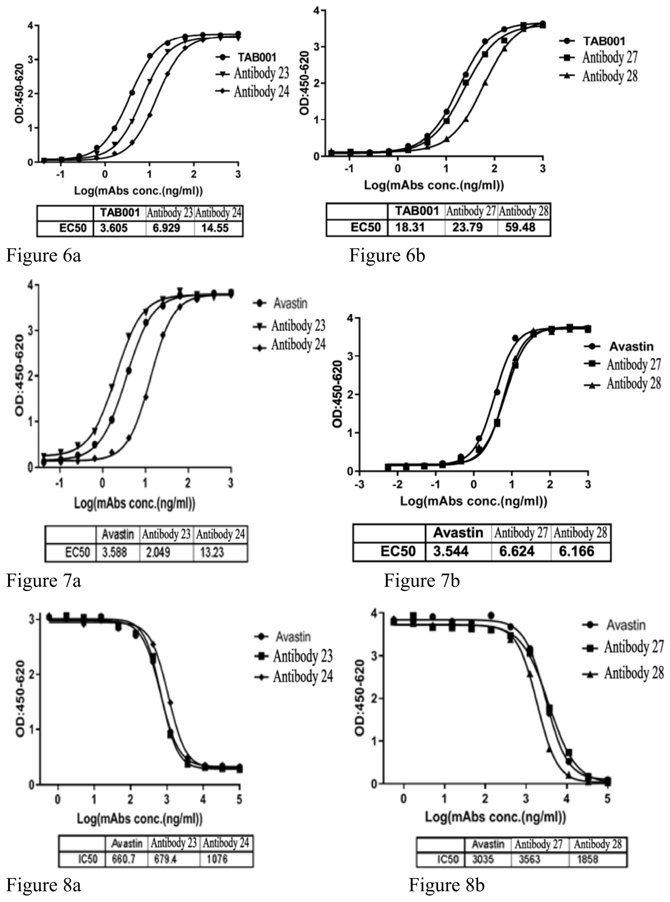

The specific results are shown in FIG. 6a, FIG. 6b, and Table 13, wherein FIG. 6a is a graph showing ELISA curves of the binding of antibody 23 and antibody 24 to human PD-1 ($EC_{50}$ of antibody 23, antibody 24 and TAB001 are 6.929 nM, 14.55 nM and 3.605 nM, respectively); FIG. 6b is a graph showing ELISA curves of the binding of antibody 27 and antibody 28 to human PD-1 ($EC_{50}$ of antibody 27, antibody 28 and TAB001 are 23.79 nM, 59.48 nM and 18.31 nM, respectively); wherein TAB001 (i.e. JS001) is the control group. As can be seen from FIG. 6a, FIG. 6b and Table 13: bispecific antibody 23, antibody 24, antibody 27 and antibody 28 all have good binding activity to human PD-1.

Table 13 Data on the binding of antibody 23, antibody 24, antibody 27 and antibody 28 molecules to human PD-1

| Sample | Antibody 23 | Antibody 24 | Antibody 27 | Antibody 28 |
|---|---|---|---|---|
| Relative binding activity (%) | 52 | 25 | 77 | 31 |
| Notes: TAB001 (JS001) data was used as a control. | | | | |

2. Detection of binding of bispecific antibody to antigen VEGFA by ELISA method

Recombinant human VEGFA (manufacturer: Sino Biological, catalog number: 11066-HNAB) was diluted to 0.5 μg/mL with PBS (manufacturer: Hyclone, catalog number: SH30256.01), and the mixture was added to the ELISA plate at 100 μL/well, and the plate was placed in a 37°C incubator and coated for 90 min; the plate was washed; 2% BSA was added to the plate at 200 μL/well, and the plate was placed in a 37°C constant temperature incubator for incubation for 90 min; the plate was washed; the samples were diluted to 1000 ng/mL with 2% BSA, followed by dilution to 0.0056 ng/mL in a 3-fold gradient. All samples were added to the ELISA plate at 100 μL/well, and the plate was placed in a 37°C constant temperature incubator for incubation for 60 min; the plate was washed; Horseradish peroxidase (HRP)-conjugated murine anti-human antibody IgG4 (manufacturer: Southern Biotech, catalog number: 9200-05) (Fc-specific) was diluted 5000-fold with 2% BSA, and the mixture was added to the ELISA plate at 100 μL/well, and the plate was placed in a 37°C constant temperature incubator for incubation for 60 min; the plate was washed; the chromogenic solution TMB (manufacturer: Sigma, catalog number: T2885) at 0.1 mg/mL was added at 100 μL/well, while avoiding bubbles, and coloration was performed at 37°C protected from light for 15 min; 2 M hydrochloric acid solution was finally added at 100 μL/well to terminate the reaction, while avoiding bubbles, the reading of microplate reader was completed within 10 min (detection wavelength: 450 nm; reference wavelength: 620 nm), and the $EC_{50}$ was obtained by fitting using the four-parameter logarithmic regression (4PL) model.

The specific results are shown in FIG. 7a, FIG. 7b, and Table 14, wherein FIG. 7a is a graph showing ELISA curves of the binding of antibody 23 and antibody 24 to human VEGFA ($EC_{50}$ of antibody 23, antibody 24 and Avastin are 2.049 nM, 13.23 nM and 3.588 nM, respectively); FIG. 7b is a graph showing ELISA curves of the binding of antibody 27 and antibody 28 to human VEGFA ($EC_{50}$ of antibody 27, antibody 28 and Avastin are 6.624 nM, 6.166 nM and 3.544 nM, respectively); wherein Avastin (bevacizumab, recombinant humanized anti-VEGF monoclonal antibody; manufacturer: Roche) is the control group. As can be seen from FIG. 7a, FIG. 7b and Table 14: bispecific antibody 23, antibody 24, antibody 27 and antibody 28 all have good binding activity to human VEGFA.

Table 14 ELISA data on the binding of antibody 23, antibody 24, antibody 27 and antibody 28 to human VEGFA

| Sample | Antibody 23 | Antibody 24 | Antibody 27 | Antibody 28 |
|---|---|---|---|---|
| Relative binding activity (%) | 175 | 27 | 53 | 57 |
| Notes: Avastin data was used as a control. | | | | |

3. Determination of the activity of bispecific antibody competing with VEGFR2 for binding to antigen VEGFA by competitive ELISA method

Recombinant human VEGFA (manufacturer: Sino Biological, catalog number: 11066-HNAB) was diluted to 0.5 μg/mL with PBS (manufacturer: Hyclone, catalog number: SH30256.01), and the mixture was added to the ELISA plate at 100 μL/well, and the plate was placed in a 37°C incubator and coated for 90 min; the plate was washed; 2% BSA was added to the plate at 200 μL/well, and the plate was placed in a 37°C constant temperature incubator for incubation for 90 min; the plate was washed; VEGFR2 His tag-biotinylated (manufacturer: Sino Biological, catalog number: 10012-H08H-B) was diluted to 0.3 μg/mL with 2% BSA, the sample was then diluted to 100000 ng/mL with VEGFR2 His tag-biotinylated at 0.3 μg/mL, followed by dilution to 0.56 ng/mL in a 3-fold gradient. All samples were added to the ELISA plate at 100 μL/well, and the plate was placed in a 37°C constant temperature incubator for incubation for 60 min; the plate was washed; horseradish peroxidase (HRP)-conjugated streptavidin (manufacturer: Jackson ImmunoResearch, catalog number: 016-030-084) was diluted 5000-fold with 2% BSA, and the mixture was added to the ELISA plate at 100 μL/well, and the plate was placed in a 37°C constant temperature incubator for incubation for 60 min; the plate was washed; the chromogenic solution TMB (manufacturer: Sigma, catalog number: T2885) at 0.1 mg/mL was added at 100 μL/well, while avoiding bubbles, and coloration was performed at 37°C protected from light for 15 min; 2 M hydrochloric acid solution was finally added at 100 μL/well to terminate the reaction, while avoiding bubbles, the reading of microplate reader was completed within 10 min (detection wavelength: 450 nm; reference wavelength: 620 nm), and the $IC_{50}$ (half-maximal inhibitory concentration) was obtained by fitting using the four-parameter logarithmic regression (4PL) model.

The specific results are shown in FIG. 8a, FIG. 8b, and Table 15, wherein FIG. 8a is a graph showing competitive inhibition ELISA curves of antibody 23 and antibody 24 blocking the binding of human VEGFR2 to VEGFA ($IC_{50}$ of antibody 23, antibody 24 and Avastin are 679.4 nM, 1076 nM and 660.7 nM, respectively); FIG. 8b is a graph showing competitive inhibition ELISA curves of antibody 27 and antibody 28 blocking the binding of human VEGFR2 to VEGFA ($IC_{50}$ of antibody 27, antibody 28 and Avastin are 3563 nM, 1858 nM and 3035 nM, respectively); wherein Avastin is the control group. As can be seen from FIG. 8a, FIG. 8b and Table 15: Bispecific antibody 23, antibody 24, antibody 27 and antibody 28 could block the binding of human VEGFR2 to human VEGFA very well.

Table 15 Competitive inhibition ELISA data of antibodies 23, 24, 27 and 28 blocking the binding of human VEGFR2 to VEGFA

| Sample | Antibody 23 | Antibody 24 | Antibody 27 | Antibody 28 |
|---|---|---|---|---|
| Relative binding activity (%) | 97 | 61 | 85 | 163 |
| Notes: Avastin data was used as a control. | | | | |

4. Determination of the activity of bispecific antibody competing with PD-L1 for binding to antigen PD-1 by competitive ELISA method

Human PD-1 (manufacturer: Suzhou Junmeng Biopharm Co., Ltd.) was diluted to 1.5 $\mu$g/mL with PBS (manufacturer: Hyclone, catalog number: SH30256.01), and the mixture was added to the ELISA plate at 100 $\mu$L/well, and the plate was placed in a 37°C incubator and coated for 90 min; the plate was washed; 2% BSA was added to the plate at 200 $\mu$L/well, and the plate was placed in a 37°C constant temperature incubator for incubation for 90 min; the plate was washed; Biotin-PD-L1 hFc was diluted to 4.0 $\mu$g/mL with 2% BSA. The sample was then diluted to 100000 ng/mL with Biotin-PD-L1 hFc at 4.0 $\mu$g/mL, followed by dilution to 0.56 ng/mL in a 3-fold gradient. All samples were added to the ELISA plate at 100 $\mu$L/well, and the plate was placed in a 37°C constant temperature incubator for incubation for 60 min; the plate was washed; horseradish peroxidase (HRP)-conjugated streptavidin (manufacturer: Jackson ImmunoResearch, catalog number: 016-030-084) was diluted 5000-fold with 2% BSA, and the mixture was added to the ELISA plate at 100 $\mu$L/well, and the plate was placed in a 37°C constant temperature incubator for incubation for 60 min; the plate was washed; the chromogenic solution TMB (manufacturer: Sigma, catalog number: T2885) at 0.1 mg/mL was added at 100 $\mu$L/well, while avoiding bubbles, and coloration was performed at 37°C protected from light for 15 min; 2 M hydrochloric acid solution was finally added at 100 $\mu$L/well to terminate the reaction, while avoiding bubbles, the reading of microplate reader was completed within 10 min (detection wavelength: 450 nm; reference wavelength: 620 nm), and the $IC_{50}$ was obtained by fitting using the four-parameter logarithmic regression (4PL) model.

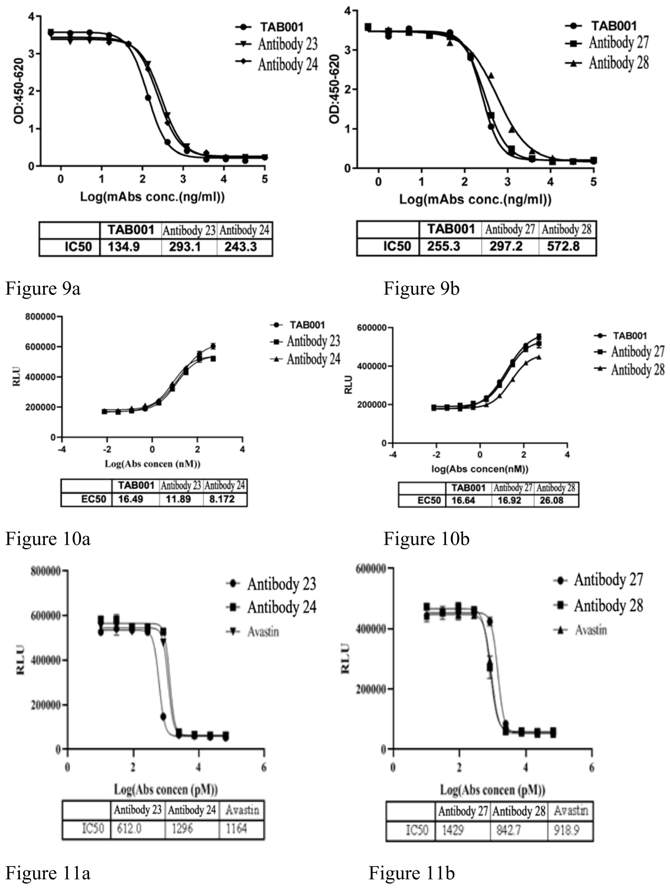

The specific results are shown in FIG. 9a, FIG. 9b, and Table 16, wherein FIG. 9a is a graph showing competitive inhibition ELISA curves of antibody 23 and antibody 24 blocking the binding of human PD-L1 to PD-1 ($IC_{50}$ of antibody 23, antibody 24 and TAB001 are 293.1 nM, 243.3 nM and 134.9 nM, respectively); FIG. 9b is a graph showing competitive inhibition ELISA curves of antibody 27 and antibody 28 blocking the binding of human PD-L1 to PD-1 ($IC_{50}$ of antibody 27, antibody 28 and TAB001 are 297.2 nM, 572.8 nM and 255.3 nM, respectively); wherein TAB001 (JS001) is the control group. As can be seen from FIG. 9a, FIG. 9b and Table 16: Bispecific antibody 23, antibody 24, antibody 27 and antibody 28 could block the binding of human PD-L1 to human PD-1 very well.

Table 16 Competitive inhibition ELISA data of antibodies 23, 24, 27 and 28 blocking the binding of human PD-L1 to PD-1

| Sample | Antibody 23 | Antibody 24 | Antibody 27 | Antibody 28 |
|---|---|---|---|---|
| Relative binding activity (%) | 46 | 55 | 86 | 45 |
| Notes: TAB001 (JS001) data was used as a control. | | | | |

## Example 5. Cellular functional activity of bispecific Antibody

1. Activity detection of bispecific antibody in PD-1/PD-L1 luciferase reporter gene system

Target cell CHO PD-L1 (stably express human PD-L1 and CD3scFv on CHO cells, purchased from Promega) cells were plated in a 96-well white flat-bottom plate at $5 \times 10^4$ cells per well overnight; after the medium in the wells containing cells was aspirated the next day, bispecific antibody or control antibody at different concentrations (starting at 500 nM, 4-fold dilution, 9 concentration gradients in total) in experimental buffer (RPMI 1640 medium ($1 \times$) + 1% FBS (fetal

bovine serum)) were added to the target cells, and the obtained system was incubated at 37°C for 30 min. Effector cells Jurkat PD-1 (stably express human PD-1 and luc2P/NFAT-RE on Jurkat cells, purchased from Promega) were then added to the cell plate at $1 \times 10^5$ cells per well and co-incubated in a 37°C incubator for 6 h; ONE-Lite luciferase detection reagent (Vazyme, Cat# 1203-03) was finally added to the cell-antibody mixed system, and the chemiluminescence signal was detected by a multifunctional microplate reader (TECAN M1000 pro). The four-parameter regression curve was fitted by GraphPad prism software, and the $EC_{50}$ value was calculated. The positive control was JS001 (recombinant humanized anti-PD-1 monoclonal antibody).

The results are shown in FIG. 10a and FIG. 10b, wherein FIG. 10a is a graph showing the results of activity detection of antibody 23 and antibody 24 in PD-1/PD-L1 luciferase reporter gene system; FIG. 10b is a graph showing the results of activity detection of antibody 27 and antibody 28 in PD-1/PD-L1 luciferase reporter gene system. As can be seen from FIG. 10a and FIG. 10b: antibody 23 (IgG4), antibody 24 (IgG4), antibody 27 (IgG4), antibody 28 (IgG4) and JS001 can effectively activate the activity of T cells in the above reporter gene system, and $EC_{50}$ of the antibodies are 11.89 nM, 8.172 nM, 16.92 nM and 26.08 nM, respectively. The activities of antibody 23 and antibody 24 are better than the activity of JS001, and the activity of antibody 27 is comparable to the activity of JS001.

2. Activity detection in a luciferase reporter gene system in which bispecific antibody block VEGF-induced NFAT signal

Target cells, i.e., 293 VEGFR2 (stably expressing VEGFR2 (KDR) and NFAT-RE-luc 2p, purchased from National Institutes for Food and Drug Control) cells were plated in 96-well white flat-bottom plate at $1 \times 10^5$ cells per well; bispecific antibody or control antibody at different concentrations (starting at 66.7 nM, 3-fold dilution, 10 concentration gradients in total) in experimental buffer (DMEM medium + 2% FBS) were added to the target cells. The VEGF antigen (Sino Biological, Cat# 11066-HNAB, working concentration of 30 ng/mL) was then added to the cell plate and co-incubated in a 37°C incubator for 5 h; ONE-Lite luciferase detection reagent (Vazyme, Cat# 1203-03) was finally added to the cell-antibody mixed system, and the chemiluminescence signal was detected by a multifunctional microplate reader (TECAN M1000 pro). The four-parameter regression curve was fitted by GraphPad prism software, and the $IC_{50}$ value was calculated. The positive control was Avastin (chemical name "bevacizumab", recombinant humanized anti-VEGF monoclonal antibody).

The results are shown in FIG. 11a and FIG. 11b, wherein FIG. 11a is a graph showing the results of activity detection in a luciferase reporter gene system in which antibody 23 and antibody 24 block VEGF-induced NFAT signal ($IC_{50}$ of antibody 23, antibody 24 and Avastin are 612 nM, 1296 nM and 1164 nM, respectively); FIG. 11b is a graph showing the results of activity detection in a luciferase reporter gene system in which antibody 27 and antibody 28 block VEGF-induced NFAT signal ($IC_{50}$ of antibody 27, antibody 28 and Avastin are 1429 nM, 842.7 nM and 918.9 nM, respectively). As can be seen from FIG. 11a and FIG. 11b: antibody 23, antibody 24, antibody 27, and antibody 28 could specifically bind to VEGF antigen, thus blocking the binding of VEGF protein to its receptor, thereby blocking downstream signaling pathways and inhibiting tumor-induced angiogenesis. The activities of antibody 24 and antibody 28 are equivalent to the activity of Avastin, and the activity of antibody 23 is better than the activity of Avastin.

3. Experiment of bispecific antibody inhibiting VEGFA-induced proliferation of HUVEC cells

VEGF antigen (working concentration of 10 ng/mL) was plated into a 96-well white plate; bispecific antibody or control antibodies at different concentrations (starting concentration of bispecific antibody at 10 nM, 3-fold dilution, 10 concentration gradients in total; starting concentration of control antibody at 66.7 nM, 3-fold dilution, 10 concentration gradients in total) in culture medium were added to a 96-well white plate and the antibodies and antigens were co-incubated in a 37°C incubator for 0.5 h. HUVEC cells (purchased from Shanghai OriBiotech Co., Ltd.) were then added into the 96-well white plate at $3 \times 10^3$ cells per well and co-incubated in a 37°C incubator for 96 h; Cell counting-Lite luciferase detection reagent (Vazyme, cat# DD1101-03) was finally added to the cell-antibody-antigen mixed system, and the chemiluminescence signal was detected by a multifunctional microplate reader (TECAN M1000 pro). The four-parameter regression curve was fitted by GraphPad prism software, and the $IC_{50}$ value was calculated. The positive control was Avastin.

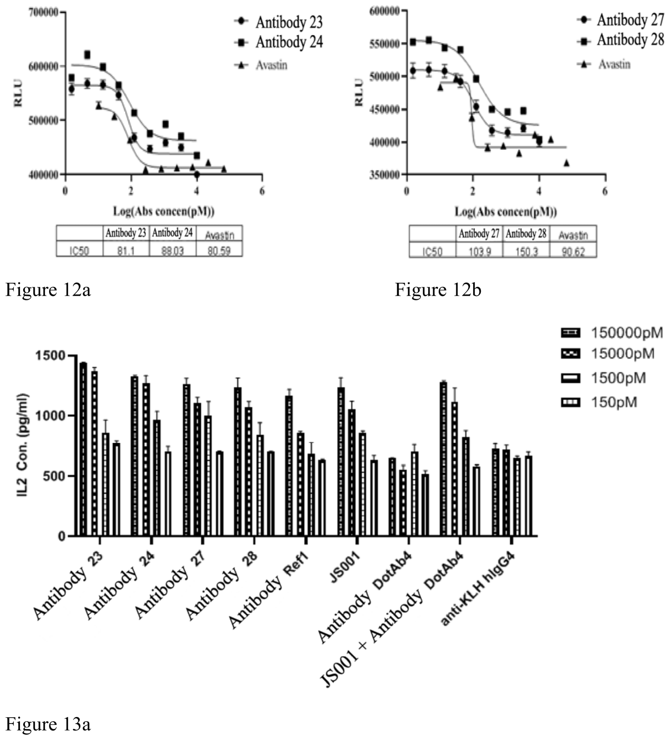

The results are shown in FIG. 12a and FIG. 12b, wherein FIG. 12a is a graph showing experimental results of antibody 23 and antibody 24 inhibiting VEGFA-induced proliferation of HUVEC cells ($IC_{50}$ of antibody 23, antibody 24 and Avastin are 81.1 nM, 88.03 nM and 80.59 nM, respectively); FIG. 12b is a graph showing experimental results of antibody 27 and antibody 28 inhibiting VEGFA-induced proliferation of HUVEC cells ($IC_{50}$ of antibody 27, antibody 28 and Avastin are 103.9 nM, 150.3 nM and 90.62 nM, respectively). As can be seen from FIG. 12a and FIG. 12b: antibody 23, antibody 24, antibody 27, and antibody 28 could specifically bind to VEGF antigen, thereby inhibiting the proliferation of HUVEC cells, and the activities of antibody 23, antibody 24, and antibody 28 are comparable to the activity of Avastin. The above results indicated that the antibodies of the present application could significantly inhibit the proliferation of HUVEC cells induced by VEGF, thereby inhibiting the angiogenesis mediated by HUVEC cells, improving tumor-induced angiogenesis, and ultimately achieving the purpose of inhibiting tumors.

4. Promotion of the secretion of cytokines IFN-γ and IL-2 in mixed lymphocyte reaction by bispecific antibody

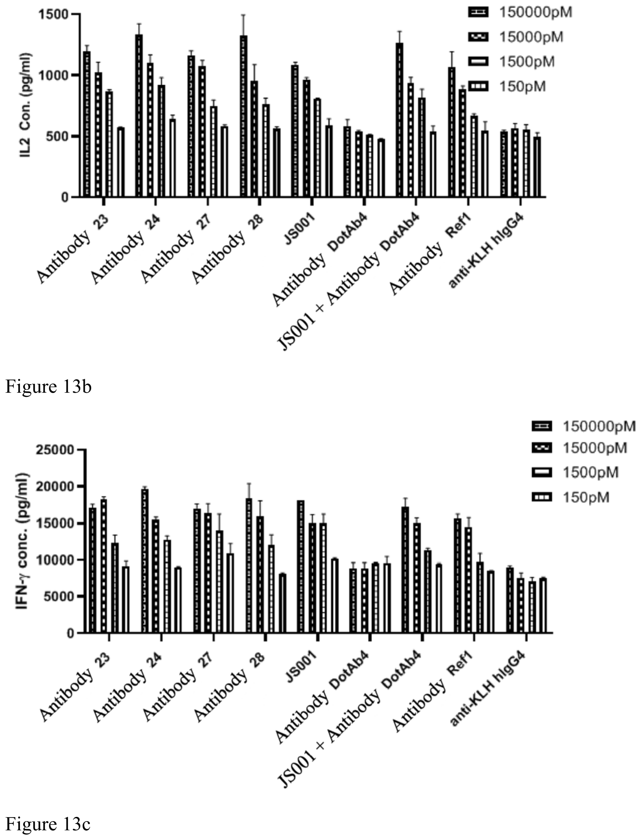

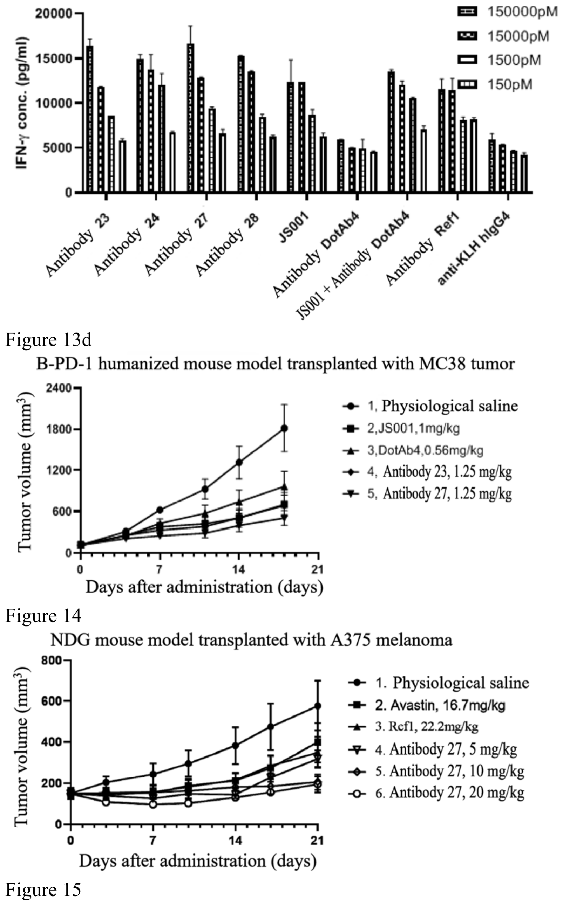

Commercial Human PBMC (purchased from Shanghai OriBiotech Co., Ltd.) cells were purified with CD14 MicroBeads, Human (Miltenyi Biotec, cat# 130-050-201) to obtain CD14 cells. Then the adherent cells were stimulated with GM-CSF (Sino Biological, cat# GMP-11846-HNAE) at 10 ng/mL and IL-4 (Sino Biological, cat# GMP-10015-HNAH) at 10 ng/mL for 5 days, respectively, followed by IL-1β (Sino Biological, cat# 10139-HNAE) at 5 ng/mL, IL-6 (Sino Biological, cat# GMP-10395-HNAE) at 10 ng/mL, TNF-α (Sino Biological, cat# GMP-10602-HNAE) at 10 ng/mL and PGE2 (dinoprostone) at 1 μmol/L for 2 days to induce mature dendritic cells (mDC, donor 1). Commercial Human PBMCs were purified with CD4T MicroBeads, Human (Miltenyi Biotec, cat# 130-045-101) to obtain CD4 T cells (donor 2).
In a 96-well round-bottom plate, the antibody to be tested at different concentrations (starting concentration of 150 nM, 10-fold dilution, 4 concentration gradients in total), mDC cells (10000 cells per well) and the above purified CD4 T cells (100000 cells per well) were co-incubated in a $CO_2$ incubator at 37°C for a total of 5 days. A portion of the reaction supernatant collected from each well of the 96-well plate on day 3 of co-incubation was detected for IL-2 release with BD CBA human IL2 Flex set (BD, cat# 558270) and was detected for the IFN-γ content in the supernatant on day 5 of co-incubation with BD CBA human IFNγ Flex set (BD, cat# 558269). The positive control was antibody Ref1 (ivonescimab, a recombinant humanized anti-PD-1 and VEGF bispecific antibody of Akeso, Inc; AK112, synthesized according to the INN published sequence (INN Number: 11809)).
The results are shown in FIG. 13a-FIG. 13d, wherein FIG. 13a is a graph showing experimental results of antibodies promoting the secretion of cytokine IL2 in donor 1 mixed lymphocyte reaction; FIG. 13b is a graph showing experimental results of antibodies promoting the secretion of cytokine IL2 in donor 2 mixed lymphocyte reaction; FIG. 13c is a graph showing experimental results of antibodies promoting the secretion of cytokine IFN-γ in donor 1 mixed lymphocyte reaction; FIG. 13d is a graph showing experimental results of antibodies promoting the secretion of cytokine IFN-γ in donor 2 mixed lymphocyte reaction. As can be seen from FIG. 13a-FIG. 13d: in the mixed lymphocyte reaction, antibody 23, antibody 24, antibody 27 and antibody 28 and the positive control antibody Ref1 could promote the release of IL-2 and IFN-γ, indicating that they could activate immune cells by abolishing the inhibition of PD-1, and restore the function of immune cells, which then kill tumor cells.

**Example 6. In vivo efficacy of bispecific antibody**

Inhibitory effect of antibody 23 and antibody 27 of the present application on B-hPD-1 mouse model transplanted with MC38.
6-week to 8-week old female B-hPD-1 humanized mice (purchased from Biocytogen Jiangsu Co., Ltd.) were inoculated subcutaneously in the right flank with $1 \times 10^6$ MC38 cells (purchased from Nanjing Cobioer Biotechnology Co., Ltd.). When the mean tumor volume was approximately 116 mm³, appropriate animals were selected and randomly divided into 5 groups according to tumor volume, with 6 animals in each group. The 5 groups were respectively: G1 physiological saline control group (solvent control group); G2 JS001 (1 mg/kg) group; G3 DotAb4 (0.56 mg/kg) group; G4 antibody 23 (1.25 mg/kg) group and G5 antibody 27 (1.25 mg/kg) group.
Intraperitoneal injection was performed. The administration was performed once every other day for 9 consecutive administrations and the experiment ended 2 days after the last administration. The tumor volume and body weight were measured twice a week, and the body weight and tumor volume of the mice were recorded. At the end of the experiment, the mice were euthanized, and the tumor inhibition rate TGI (%) was calculated, TGI (%) = [1-(Ti-T0)/(Vi-V0)] × 100% (Ti: the mean tumor volume of the treatment group on day i of administration, T0: the mean tumor volume of the treatment group on day 0 of administration; Vi: the mean tumor volume of the solvent control group on day i of administration, and V0: the mean tumor volume of the solvent control group on day 0 of administration).
The results are shown in Table 17 and FIG. 14, wherein FIG. 14 is a graph showing the inhibitory effect of a test substance on a B-PD-1 humanized mouse model transplanted with MC38 tumor, and Table 17 is a table showing the efficacy analysis of each group in the B-PD-1 humanized mouse model transplanted with MC38 tumor (day 18 after the start of administration). As can be seen from Table 17 and FIG. 14: the mean tumor volume of the physiological saline control group was 1815 ± 98.7 mm³ on day 18 after administration. The mean tumor volume of the JS001 (1 mg/kg) group was 702 ± 7.72 mm³, and the tumor inhibition rate was 65.5%, significantly inhibiting tumor growth compared with the physiological saline control group; the mean tumor volume of the DotAb4 (0.56 mg/kg) group was 968 ± 15.01 mm³, and the tumor inhibition rate was 49.9%, showing a slight tumor inhibitory effect compared with the physiological saline control group. The mean tumor volume of the antibody 23 (1.25 mg/kg) and antibody 27 (1.25 mg/kg) groups was 687 mm³ and 505 mm³, respectively, and the tumor inhibition rate was 66.4% and 77.1%, respectively, significantly inhibiting tumor growth compared with the physiological saline control group.

Table 17 Efficacy analysis table of each group in the B-PD-1 humanized mouse model transplanted with MC38 tumor

| Experimental group | Tumor Volume ($\bar{x} \pm$S) | TGI (%) | P value (compared with the control group) |
|---|---|---|---|
| 1, Physiological saline | 1815±98.7 | - | - |
| 2, JS001, 1 mg/kg | 702±7.72 | 65.5 | 0.016 |
| 3, DotAb4, 0.56 mg/kg | 968±15.01 | 49.9 | 0.061 |
| 4, Antibody 23, 1.25 mg/kg | 687±0 | 66.4 | 0.013 |
| 5, Antibody 27, 1.25 mg/kg | 505±0 | 77.1 | 0.004 |
| Notes: 1. The data is expressed as "mean ± standard error", and "-" indicates absence; 2. $$TGI\% = [1\text{-}(Ti\text{-}T0)/(Vi\text{-}V0)] \times 100\%;$$ 3. The P value was obtained by comparing the tumor volume of each group by the T-test method. | | | |

## Example 7. In vivo efficacy of bispecific antibody

Inhibitory effect of antibody 27 of the present application on NDG mouse model transplanted with A375 melanoma.

A375 human malignant melanoma cells and Matrigel were mixed evenly at a 1 : 1 volume ratio. 0.2 mL of the mixture (containing $5 \times 10^6$ A375 human malignant melanoma cells) was taken and inoculated subcutaneously on the right back of NDG mice (purchased from Biocytogen Jiangsu Co., Ltd.). When the tumor grew to about 137 mm$^3$, 0.2 mL (containing $10 \times 10^6$ human PBMC peripheral blood mononuclear cells, purchased from Shanghai OriBiotech Co., Ltd., catalog number: LP220829009) was inoculated via the tail vein. Two days later, 24 mice were selected and randomly grouped according to their tumor volume, with 4 mice in each group, totaling 6 groups. The groups were respectively: G1 physiological saline control group, G2 Avastin (16.7 mg/kg), G3 Ref1 (22.2 mg/kg), G4 antibody 27 (5 mg/kg), G5 antibody 27 (10 mg/kg) group and G6 antibody 27 (20 mg/kg).

All animals were administered by intraperitoneal injection twice a week for a total of 6 administrations. The tumor volume and body weight were measured twice a week, and the body weight and tumor volume of the mice were recorded. At the end of the experiment (i.e., day 21 after administration), the mice were euthanized, and the tumor inhibition rate TGI (%) was calculated, TGI (%) = $[1\text{-}(Ti\text{-}T0)/(Vi\text{-}V0)] \times 100\%$ (Ti: the mean tumor volume of the treatment group on day i of administration, T0: the mean tumor volume of the treatment group on day 0 of administration; Vi: the mean tumor volume of the solvent control group on day i of administration, and V0: the mean tumor volume of the solvent control group on day 0 of administration).

The results are shown in Table 18 and FIG. 15, wherein FIG. 15 is a graph showing the inhibitory effect of the test substance on an NDG immunodeficient mouse model transplanted with A375 tumor, and Table 18 is a table showing efficacy analysis of each group in the NDG immunodeficient mouse model transplanted with A375 tumor (day 21 after the start of administration). As can be seen from Table 18 and FIG. 15: the mean tumor volume of the G2 Avastin (16.7 mg/kg) group was 398 ± 164 mm$^3$, and the tumor inhibition rate was 41.7%. The mean tumor volume of the G3 Ref1 (22.2 mg/kg) group was 350 ± 149.2 mm$^3$, and the tumor inhibition rate was 53.0%. The mean tumor volume of the G4 antibody 27 (5 mg/kg) group was: 321 ± 79.3 mm$^3$, and the tumor inhibition rate was 59.8%. The mean tumor volume of the G5 antibody 27 (10 mg/kg) group was 206 ± 76.7 mm$^3$, and the tumor inhibition rate was 86.7%. The mean tumor volume of the G6 antibody 27 (20 mg/kg) group was 195 ± 80.1 mm$^3$, and the tumor inhibition rate was 89.0%. In addition, the efficacy of G6 antibody 27 (20 mg/kg) was better than that of G3 Ref1 (22.2 mg/kg), and significantly inhibited tumor growth compared with physiological saline group (P < 0.05).

Table 18 Efficacy analysis table of each group in the NDG immunodeficient mouse model transplanted with A375 tumor

| Experimental group | Tumor Volume ($\bar{x} \pm$S) | TGI (%) | P value (compared with the control group) |
|---|---|---|---|
| 1, Physiological saline | 577±243.7 | - | - |
| 2, Avastin, 16.7 mg/kg | 398±164.0 | 41.7 | 0.326 |
| 3, Ref 1, 22.2 mg/kg | 350±149.2 | 53.0 | 0.163 |
| 4, Antibody 27, 5 mg/kg | 321±79.3 | 59.8 | 0.092 |
| 5, Antibody 27, 10 mg/kg | 206±76.7 | 86.7 | 0.027 |

(continued)

| Experimental group | Tumor Volume ($\bar{x} \pm S$) | TGI (%) | P value (compared with the control group) |
|---|---|---|---|
| 6, antibody 27, 20 mg/kg | 195±80.1 | 89.0 | 0.025 |

Notes: 1. The data is expressed as "mean ± standard error", and "-" indicates absence; 2.

$$TGI\% = [1-(Ti-T0)/(Vi-V0)] \times 100\%;$$

3. The P value was obtained by comparing the tumor volume of each group by the T-test method.

## Example 8. Pharmacokinetics of bispecific antibody in cynomolgus monkey

Female cynomolgus monkeys (animal source: Guangxi Fangchenggang Changchun Biotechnology Development Co., Ltd.; license number for use: SCXK Gui 2018-0005) (two monkeys in each group) was administered with bispecific antibodies (antibody Ref1 (ivonescimab, recombinant humanized anti-PD-1 and VEGF bispecific antibody of Akeso, Inc, AK112), antibody 23 and antibody 27) with different codes via a single intravenous drip for 5 min at a dose of 3 mg/kg, and about 1 mL of whole blood samples were collected at 0 min (before administration), 5 min (end of infusion), 1 h, 6 h, 24 h, 48 h, 72 h, 144 h, 168 h, 240 h, 336 h, and 504 h. After the whole blood sample was naturally coagulated, the serum was separated and stored for blood drug concentration detection. The specific detection method is as follows:

Human/Cynomolgus VEGF/VEGFA/VEGF165 Protein (Sino Biological, 11066-HNAB) was diluted to 0.3 $\mu$g/mL with PBS, and a 96-well plate was coated with the obtained mixture at 100 $\mu$L/well, blocked with 2 wt% BSA. Standard curve (4000 ng/mL (anchor point), 2000 ng/mL, 1000 ng/mL, 500 ng/mL, 250 ng/mL, 125 ng/mL, 62.5 ng/mL, 31.3 ng/mL and 15.6 ng/mL (anchor point)), quality control points (HQC (1600 ng/mL), MQC (400 ng/mL) and LQC (100 ng/mL)) and samples to be tested (diluted to the standard curve range) were diluted 20-fold with 2 wt% BSA and then added to the plate at 100 $\mu$L/well. Then, 0.2 $\mu$g/mL human PD-1 his tag (prepared in-house) diluted in 2 wt% BSA was added to the plate at 100 $\mu$L/well. The detection antibody was anti-his tag antibody (HRP) diluted with 2 wt% BSA to 0.1 $\mu$g/mL and added to the plate at 100 $\mu$L/well. Tetramethylbenzidine (TMB) was then added at 100 $\mu$L/well and the reaction was finally terminated with 2 M hydrochloric acid. The absorbance (OD value) was measured with a microplate reader at a wavelength of 450 nm/620 nm, and the sample concentration was calculated with SoftMax Pro 5.4.1 software.

The drug concentrations and pharmacokinetic parameters in the serum of each group of animals are summarized in Table 19 and Table 20 below, wherein $AUC_{(0-t)}$ refers to the area enclosed by the blood drug concentration curve against the time axis; $t_{1/2z}$ refers to the terminal blood drug concentration and half-life; $T_{max}$ refers to the time to reach peak, i.e., the time required to reach the peak concentration of the drug after administration; $V_z$ refers to the apparent volume of distribution, i.e., the proportional constant between the amount of drug in the body and the blood drug concentration when the drug reaches dynamic equilibrium in the body; $Cl_z$ refers to clearance rate; $C_{max}$ refers to the drug peak concentration, i.e., the highest blood drug concentration after administration.

Table 19 Summary of drug concentrations in serum of each group

| Concentration ($\mu$g/mL) | Antibody ref1 (Akeso) | | Antibody 23 | | Antibody 27 | |
|---|---|---|---|---|---|---|
| Time (h) | AF01 | AF02 | BF01 | BF02 | CF01 | CF02 |
| 0 | BLQ | BLQ | BLQ | BLQ | BLQ | BLQ |
| 0.083 | 102.8 | 64.8 | 102.5 | 107.5 | 120.7 | 79.8 |
| 1 | 99.8 | 60.3 | 96.7 | 102.1 | 86.6 | 77.1 |
| 6 | 64.0 | 34.9 | 73.9 | 79.5 | 68.8 | 38.0 |
| 24 | 34.8 | 21.9 | 57.1 | 32.4 | 43.6 | 20.9 |
| 48 | 25.1 | 14.5 | 33.3 | 12.7 | 35.6 | 6.3 |
| 72 | 12.5 | 8.5 | 18.7 | 4.9 | 27.7 | 1.6 |
| 144 | 3.4 | 3.7 | 1.9 | 0.3 | 16.9 | 0.1 |
| 168 | 2.7 | 2.0 | 2.0 | 0.2 | 13.7 | BLQ |
| 240 | 0.4 | 0.5 | 0.4 | BLQ | 3.1 | BLQ |
| 336 | BLQ | BLQ | BLQ | BLQ | 0.2 | BLQ |
| 504 | BLQ | BLQ | BLQ | BLQ | BLQ | BLQ |

(continued)

| Concentration ($\mu$g/mL) | Antibody ref1 (Akeso) | Antibody 23 | Antibody 27 |
|---|---|---|---|
| Notes: 1) The lower limit of quantification of the method is 31.3 ng/mL 2) BLQ is below the lower limit of quantification | | | |

Table 20 Pharmacokinetic parameters of each group

| Intravenous drip (3 mg/kg) | | Antibody ref1 (Akeso) | | Antibody 23 | | Antibody 27 | |
|---|---|---|---|---|---|---|---|
| Parameter | Unit | mean | SD | mean | SD | mean | SD |
| $AUC_{(0-t)}$ | $\mu$g/mL$\times$d | 113.5 | 25.1 | 141.7 | 37.0 | 152.7 | 95.2 |
| $t_{1/2z}$ | d | 1.6 | 0.2 | 1.0 | 0.2 | 0.9 | 0.3 |
| $T_{max}$ | d | 0.003 | 0 | 0.003 | 0 | 0.003 | 0 |
| $V_z$ | mL/kg | 64 | 21 | 30.5 | 0.5 | 34.5 | 13.5 |
| $Cl_z$ | mL/d/kg | 27.5 | 5.5 | 23.0 | 6.0 | 32.0 | 20.0 |
| $C_{max}$ | $\mu$g/mL | 83.8 | 19.0 | 105.0 | 2.5 | 100.3 | 20.5 |

As can be seen from Table 20: After a single intravenous drip of the test drug in female cynomolgus monkeys at a dose of 3 mg/kg, the AUC(0-t) of antibody 27, antibody 23, and antibody ref1 (Akeso) were 152.7 $\pm$ 95.2 $\mu$g/mL $\times$ d, 141.7 $\pm$ 37.0 $\mu$g/mL $\times$ d, and 113.5 $\pm$ 25.1 $\mu$g/mL $\times$ d, respectively, indicating that high exposure levels could be achieved after administration, with half-life $t_{1/2z}$ of 0.9 $\pm$ 0.3 days, 1.0 $\pm$ 0.2 days, and 1.6 $\pm$ 0.2 days, respectively, which were equivalent to the elimination efficiency of conventional bispecific antibodies, indicating that this drug has good druggability. $V_z$ of antibody 27 and antibody 23 was about 20-50 mL/kg, indicating that this drug was mainly in the circulation after intravenous injection in monkeys and no extravascular distribution occurred.

The above description is only a preferred example of the present application, and is not intended to limit the present application. Any modifications, equivalent substitutions, improvements, etc. made within the spirit and principles of the present application shall be included within the scope of protection of the present application.

**Claims**

1. A bispecific antibody comprising a first protein functional region and a second protein functional region; wherein: the first protein functional region is an anti-PD-1 antibody or an antigen-binding fragment thereof, and the second protein functional region is an anti-VEGF antibody or an antigen-binding fragment thereof, wherein the anti-VEGF antibody or the antigen-binding fragment thereof comprises a heavy chain variable region comprising HCDR1, HCDR2, and HCDR3 with amino acid sequences as set forth in SEQ ID NO: 7, SEQ ID NO: 8, and SEQ ID NO: 9, respectively.

2. The bispecific antibody according to claim 1, wherein the anti-PD-1 antibody or the antigen-binding fragment thereof comprises a heavy chain variable region and/or a light chain variable region; the amino acid sequences of HCDR1, HCDR2, and HCDR3 comprised in the heavy chain variable region are selected from the group consisting of the amino acid sequences of HCDR1, HCDR2, and HCDR3 comprised in the heavy chain variable region of nivolumab, pembrolizumab, cemiplimab, toripalimab, sintilimab, or camrelizumab, respectively; the amino acid sequences of LCDR1, LCDR2, and LCDR3 comprised in the light chain variable region are selected from the group consisting of the amino acid sequences of LCDR1, LCDR2, and LCDR3 comprised in the light chain variable region of nivolumab, pembrolizumab, cemiplimab, toripalimab, sintilimab, or camrelizumab, respectively.

3. The bispecific antibody according to claim 1 or 2, wherein the anti-PD-1 antibody or the antigen-binding fragment thereof comprises a heavy chain variable region comprising HCDR1, HCDR2 and HCDR3 with amino acid sequences as set forth in SEQ ID NO: 1, SEQ ID NO: 2 and SEQ ID NO: 3, respectively, and/or a light chain variable region comprising LCDR1, LCDR2 and LCDR3 with amino acid sequences as set forth in SEQ ID NO: 4, SEQ ID NO: 5 and SEQ ID NO: 6, respectively.

4. The bispecific antibody according to any one of claims 1-3, wherein

the anti-PD-1 antibody or the antigen-binding fragment thereof comprises: a heavy chain variable region having at least 90%, 91%, 92%, 93%, 94%, 95%, 96%, 97%, 98%, 99%, or 100% sequence identity to an amino acid sequence as set forth in SEQ ID NO: 10, and a light chain variable region having at least 90%, 91%, 92%, 93%, 94%, 95%, 96%, 97%, 98%, 99%, or 100% sequence identity to an amino acid sequence as set forth in SEQ ID NO: 11; and/or

the anti-VEGF antibody or the antigen-binding fragment thereof comprises: a heavy chain variable region having at least 90%, 91%, 92%, 93%, 94%, 95%, 96%, 97%, 98%, 99% or 100% sequence identity to an amino acid sequence as set forth in SEQ ID NO: 12.

5. The bispecific antibody according to any one of claims 1-3, wherein

the anti-PD-1 antibody or the antigen-binding fragment thereof is selected from the group consisting of Fab, Fab', F(ab')2, Fd, Fv, dAb, a complementary determining region fragment, a single-domain antibody, a single chain antibody, a humanized antibody, a chimeric antibody and a diabody; and/or

the anti-VEGF antibody or the antigen-binding fragment thereof is selected from the group consisting of Fab, Fab', F(ab')2, Fd, Fv, dAb, a complementary determining region fragment, a single-domain antibody, a single chain antibody, a humanized antibody, a chimeric antibody and a diabody.

6. The bispecific antibody according to any one of claims 1-5, wherein

the first protein functional region is an immunoglobulin, and/or
the second protein functional region is a single-domain antibody.

7. The bispecific antibody according to claim 6, wherein the immunoglobulin is selected from the group consisting of IgG, IgA, IgD, IgE and IgM; preferably IgG.

8. The bispecific antibody according to claim 6, wherein

a constant region of the immunoglobulin is derived from a human antibody;
preferably, the constant region of the immunoglobulin is selected from the group consisting of a constant region of human IgG1, IgG2, IgG3 or IgG4.

9. The bispecific antibody according to claim 6, wherein the single-domain antibodies are two in number, and one terminus of each of the single-domain antibodies is respectively connected to the C-terminus or N-terminus of two heavy chains of the immunoglobulin; or

one terminus of each of the single-domain antibodies is respectively connected to the C-terminus or N-terminus of two light chains of the immunoglobulin; or

the N-terminus of each of the single-domain antibodies is respectively connected to the C-terminus of the heavy chain constant region CH1 of the immunoglobulin, and the C-terminus of each of the single-domain antibodies is respectively connected to the N-terminus of the heavy chain constant region CH2 of the immunoglobulin.

10. The bispecific antibody according to claim 9, wherein

(1) the first polypeptide chain of the bispecific antibody is represented by a formula VLPD-1-CL, and the second polypeptide chain is represented by a formula VHVEGF-linker-VHPD-1-CH1-hinge-CH2-CH3; or
(2) the first polypeptide chain of the bispecific antibody is represented by a formula VLPD-1-CL, and the second polypeptide chain is represented by a formula VHPD-1-CH1-hinge-CH2-CH3-linker-VHVEGF; or
(3) the first polypeptide chain of the bispecific antibody is represented by a formula VLPD-1-CL, and the second polypeptide chain is represented by a formula VHPD-1-CH1-linker-VHVEGF-linker-hinge-CH2-CH3; or
(4) the first polypeptide chain of the bispecific antibody is represented by a formula VHVEGF-linker-VLPD-1-CL, and the second polypeptide chain is represented by a formula VHPD-1-CH1-hinge-CH2-CH3.

11. The bispecific antibody according to claim 10, wherein

(1) the first polypeptide chain comprises an amino acid sequence as set forth in SEQ ID NO: 13, and the second polypeptide chain comprises an amino acid sequence as set forth in SEQ ID NO: 14; or
(2) the first polypeptide chain comprises an amino acid sequence as set forth in SEQ ID NO: 13, and the second polypeptide chain comprises an amino acid sequence as set forth in SEQ ID NO: 15; or
(3) the first polypeptide chain comprises an amino acid sequence as set forth in SEQ ID NO: 13, and the second polypeptide chain comprises an amino acid sequence as set forth in SEQ ID NO: 16; or
(4) the first polypeptide chain comprises an amino acid sequence as set forth in SEQ ID NO: 17, and the second polypeptide chain comprises an amino acid sequence as set forth in SEQ ID NO: 18.

12. An isolated nucleic acid molecule encoding the bispecific antibody according to any one of claims 1 to 11.

13. A vector comprising the isolated nucleic acid molecule according to claim 12.

14. A host cell comprising the isolated nucleic acid molecule according to claim 12, or the vector according to claim 13.

15. A method for preparing the bispecific antibody according to any one of claims 1 to 11, comprising the steps of culturing the host cell according to claim 14, and obtaining the bispecific antibody from the culture.

16. A conjugate comprising the bispecific antibody according to any one of claims 1 to 11, and a conjugation moiety, wherein the conjugation moiety is a therapeutically active substance or a detectable label; preferably, the therapeutically active substance is a cytotoxin, a cytokine or a radioactive nuclide; preferably, the detectable label is selected from the group consisting of a radioisotope, a fluorescent substance, a luminescent substance, a chromogenic substance, and an enzyme.

17. A kit comprising the bispecific antibody according to any one of claims 1 to 11, or comprising the conjugate according to claim 16;
preferably, the kit further comprises a second antibody capable of specifically binding to the bispecific antibody; preferably, the second antibody further comprises a detectable label, more preferably, the detectable label is selected from the group consisting of a radioisotope, a fluorescent substance, a luminescent substance, a chromogenic substance and an enzyme.

18. A pharmaceutical composition comprising the bispecific antibody according to any one of claims 1 to 11, or comprising the conjugate according to claim 16; preferably, the pharmaceutical composition further comprises a pharmaceutically acceptable excipient.

19. A method for treating or ameliorating a malignant tumor, comprising administering to a patient in need thereof the bispecific antibody according to any one of claims 1 to 11, or the conjugate according to claim 16, or the pharmaceutical composition according to claim 18, wherein the malignant tumor is a PD-L1 and/or VEGF overexpressing tumor; preferably, the malignant tumor is a solid tumor; more preferably, the malignant tumor is lung cancer, colon cancer, colorectal cancer, melanoma or ovarian cancer; more preferably, the lung cancer is non-small cell lung cancer or small cell lung cancer; more preferably, the non-small cell lung cancer is squamous non-small cell lung cancer or non-squamous non-small cell lung cancer; more preferably, the non-squamous non-small cell lung cancer is locally advanced or metastatic non-squamous non-small cell lung cancer.

20. A method for modulating an immune response and modulating tumor angiogenesis, comprising administering to a patient in need thereof the bispecific antibody according to any one of claims 1 to 11, or the conjugate according to claim 16, or the pharmaceutical composition according to claim 18; preferably, the modulating the immune response is achieved by blocking the binding of PD-1 to a receptor thereof, and the modulating tumor angiogenesis is achieved by blocking the binding of VEGF to a receptor thereof.

Figure 1

Figure 2a

Figure 2b

Figure 2c

Figure 2d

Figure 3a

Figure 3b

Figure 3c

Figure 3d

Figure 3e

Figure 4a

Figure 4b

Figure 4c

Figure 4d

Figure 4e

Figure 5a

Figure 5b

Figure 5c

|  | TAB001 | Antibody 23 | Antibody 24 |
|---|---|---|---|
| EC50 | 3.605 | 6.929 | 14.55 |

Figure 6a

|  | TAB001 | Antibody 27 | Antibody 28 |
|---|---|---|---|
| EC50 | 18.31 | 23.79 | 59.48 |

Figure 6b

|  | Avastin | Antibody 23 | Antibody 24 |
|---|---|---|---|
| EC50 | 3.588 | 2.049 | 13.23 |

Figure 7a

|  | Avastin | Antibody 27 | Antibody 28 |
|---|---|---|---|
| EC50 | 3.544 | 6.624 | 6.166 |

Figure 7b

|  | Avastin | Antibody 23 | Antibody 24 |
|---|---|---|---|
| IC50 | 660.7 | 679.4 | 1076 |

Figure 8a

|  | Avastin | Antibody 27 | Antibody 28 |
|---|---|---|---|
| IC50 | 3035 | 3563 | 1858 |

Figure 8b

Figure 9a

Figure 9b

Figure 10a

Figure 10b

Figure 11a

Figure 11b

Figure 12a

Figure 12b

Figure 13a

Figure 13b

Figure 13c

Figure 13d

B-PD-1 humanized mouse model transplanted with MC38 tumor

Figure 14

NDG mouse model transplanted with A375 melanoma

Figure 15

## INTERNATIONAL SEARCH REPORT

| International application No. |
|---|
| **PCT/CN2024/094648** |

| A. | CLASSIFICATION OF SUBJECT MATTER |
|---|---|

C07K16/46(2006.01)i; C12N15/13(2006.01)i; G01N33/68(2006.01)i; A61K39/395(2006.01)i; A61P35/00(2006.01)i; A61P35/02(2006.01)i

According to International Patent Classification (IPC) or to both national classification and IPC

| B. | FIELDS SEARCHED |
|---|---|

Minimum documentation searched (classification system followed by classification symbols)

IPC: C07K C12N G01N A61K A61P

Documentation searched other than minimum documentation to the extent that such documents are included in the fields searched

Electronic data base consulted during the international search (name of data base and, where practicable, search terms used)

CNTXT; VCN; VEN; ENTXT; ENTXTC; OETXT; CNKI; 万方, WANFANG; 读秀学术, Duxiu Scholar; 中国专利生物序列检索系统, China Patent Biological Sequence Search System; NCBI; STNEXT; ISI Web of Science; Springer; 上海君实生物, JUNSHI BIOSCIENCES, 双特异性抗体, PD-1, VEGF, 重链可变区, 轻链可变区, CDR, IgG, 偶联物, 肿瘤, 癌症, Heavy Chain Variable Region, Light Chain Variable Region, conjugate, tumor, cancer, 基于序列1-18的检索, search based on sequences 1-18

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | CN 109053895 A (AKESO BIOPHARMA, INC.) 21 December 2018 (2018-12-21) claims 1-22 | 1-10, 12-20 |
| A | CN 109053895 A (AKESO BIOPHARMA, INC.) 21 December 2018 (2018-12-21) claims 1-22 | 11 |
| Y | WO 2022175474 A1 (DOTBIO PTE. LTD. et al.) 25 August 2022 (2022-08-25) claims 1, 5 and 9, and description, page 38, sequence 1 | 1-10, 12-20 |
| A | WO 2022175474 A1 (DOTBIO PTE. LTD. et al.) 25 August 2022 (2022-08-25) claims 1, 5 and 9, and description, page 38, sequence 1 | 11 |
| Y | CN 115006517 A (SHANGHAI JUNSHI BIOSCIENCES CO., LTD. et al.) 06 September 2022 (2022-09-06) abstract, and description, paragraphs [0171]-[0189] | 1-10, 12-20 |

| ☑ Further documents are listed in the continuation of Box C. | ☑ See patent family annex. |
|---|---|

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **14 August 2024** | **21 August 2024** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

International application No.

**PCT/CN2024/094648**

### C. DOCUMENTS CONSIDERED TO BE RELEVANT

| Category* | Citation of document, with indication, where appropriate, of the relevant passages | Relevant to claim No. |
|---|---|---|
| A | CN 115006517 A (SHANGHAI JUNSHI BIOSCIENCES CO., LTD. et al.) 06 September 2022 (2022-09-06) abstract, and description, paragraphs [0171]-[0189] | 11 |
| A | CN 113244386 A (SHANGHAI JUNSHI BIOSCIENCES CO., LTD.) 13 August 2021 (2021-08-13) claims 1-15 | 1-20 |

Form PCT/ISA/210 (second sheet) (July 2022)

# EP 4 717 714 A1

| INTERNATIONAL SEARCH REPORT | International application No. |
| --- | --- |
| | **PCT/CN2024/094648** |

| Box No. I        Nucleotide and/or amino acid sequence(s) (Continuation of item 1.c of the first sheet) |
| --- |

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

       ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
| --- |
| **PCT/CN2024/094648** |

| Box No. II      Observations where certain claims were found unsearchable (Continuation of item 2 of first sheet) |
| --- |

This international search report has not been established in respect of certain claims under Article 17(2)(a) for the following reasons:

1. ☑ Claims Nos.: **19, 20**
because they relate to subject matter not required to be searched by this Authority, namely:

The methods of claims 19 and 20 are both methods for treatment of a living human or animal body, which fall within subject matter for which no search is required by the International Searching Authority as defined in PCT Rule 39.1.

The subject matter of claims 19 and 20 is reasonably expected to be amended as follows:

Claim 19: the use of the bispecific antibody according to any one of claims 1 to 11 or the conjugate of claim 16 or the pharmaceutical composition of claim 18 in the preparation of a product for treating or ameliorating malignant tumors, ...

Claim 20: the use of the bispecific antibody according to any one of claims 1 to 11 or the conjugate of claim 16 or the pharmaceutical composition of claim 18 in the preparation of a product for regulating immune response and regulating tumor angiogenesis.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

International application No.

**PCT/CN2024/094648**

| Patent document cited in search report | | | Publication date (day/month/year) | Patent family member(s) | | | Publication date (day/month/year) |
|---|---|---|---|---|---|---|---|
| CN | 109053895 | A | 21 December 2018 | JP | 2024023186 | A | 21 February 2024 |
| | | | | MX | 2021002312 | A | 02 July 2021 |
| | | | | AU | 2019332708 | A1 | 15 April 2021 |
| | | | | SG | 11202101985 | TA | 29 April 2021 |
| | | | | US | 2021340239 | A1 | 04 November 2021 |
| | | | | JP | 2021536242 | A | 27 December 2021 |
| | | | | JP | 7374995 | B2 | 07 November 2023 |
| | | | | PH | 12021550407 | A1 | 20 September 2021 |
| | | | | ZA | 202101894 | B | 31 August 2022 |
| | | | | CA | 3110749 | A1 | 05 March 2020 |
| | | | | BR | 112021003559 | A2 | 25 May 2021 |
| | | | | EA | 202190535 | A1 | 20 July 2021 |
| | | | | US | 2023340097 | A1 | 26 October 2023 |
| | | | | KR | 20210053912 | A | 12 May 2021 |
| | | | | WO | 2020043184 | A1 | 05 March 2020 |
| | | | | EP | 3882275 | A1 | 22 September 2021 |
| | | | | EP | 3882275 | A4 | 10 August 2022 |
| | | | | IL | 281116 | A | 29 April 2021 |
| WO | 2022175474 | A1 | 25 August 2022 | JP | 2024512260 | A | 19 March 2024 |
| | | | | AR | 124917 | A1 | 17 May 2023 |
| | | | | JP | 2024513644 | A | 27 March 2024 |
| | | | | EP | 4294839 | A1 | 27 December 2023 |
| | | | | US | 2024132580 | A1 | 25 April 2024 |
| | | | | US | 2024228602 | A9 | 11 July 2024 |
| | | | | CA | 3208368 | A1 | 25 August 2022 |
| | | | | AU | 2022223337 | A1 | 21 September 2023 |
| | | | | CA | 3208389 | A1 | 25 August 2022 |
| | | | | US | 2024132579 | A1 | 25 April 2024 |
| | | | | US | 2024228601 | A9 | 11 July 2024 |
| | | | | KR | 20230165901 | A | 05 December 2023 |
| | | | | KR | 20230165902 | A | 05 December 2023 |
| | | | | EP | 4294838 | A1 | 27 December 2023 |
| | | | | WO | 2022175481 | A1 | 25 August 2022 |
| | | | | AU | 2022222311 | A1 | 21 September 2023 |
| CN | 115006517 | A | 06 September 2022 | WO | 2022184148 | A1 | 09 September 2022 |
| CN | 113244386 | A | 13 August 2021 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2023105756067
- CN 117242092 A
- US 4816567 P

**Non-patent literature cited in the description**

- **HOMET M.B.** ; **PARISI G. et al.** Anti-PD-1 Therapy in Melanoma. *SeminOncol.*, June 2015, vol. 42 (3), 466-473
- **HELD SA** ; **HEINE A et al.** Advancesin immunotherapy of chronic myeloid leukemia CML. *Curr Cancer Drug Targets*, September 2013, vol. 13 (7), 768-74
- **COLOMA MJ** ; **MORRISON SL.** Design and production of novel tetravalent bispecific antibodies. *Nat Biotechnol. Nature Biotechnology*, 1997, vol. 15, 159-163
- **MILLER BR** ; **DEMAREST SJ et al.** Stability engineering of scFvs for the development of bispecific and multivalent antibodies. *Protein Eng Des Sel*, 2010, vol. 23, 549-57
- **FITZGERALD J** ; **LUGOVSKOY A.** Rational engineering of antibody therapeutics targeting multiple oncogene pathways. *MAbs*, 2011, vol. 3, 299-309
- **KABAT.** Sequences of Proteins of ImmunologicaIInterest. National Institutes of Health, 1987
- **CHOTHIA** ; **LESK.** *J. Mol. Biol.*, 1987, vol. 196, 901-917
- **CHOTHIA et al.** *Nature*, 1989, vol. 342, 878-883
- Fundamental Immunology. Raven Press, 1989
- **WARD et al.** *Nature*, 1989, vol. 341, 544-546
- **BIRD et al.** *Science*, 1988, vol. 242, 423-426
- **HUSTON et al.** *Proc. Natl. Acad. Sci. USA*, 1988, vol. 85, 5879-5883
- **HOLLIGER et al.** *Proc. Natl. Acad. Sci. USA*, 1993, vol. 90, 6444-6448
- **ALFTHAN et al.** *Protein Eng.*, 1995, vol. 8, 725-731
- **CHOI et al.** *Eur. J. Immunol.*, 2001, vol. 31, 94-106
- **HU et al.** *Cancer Res.*, 1996, vol. 56, 3055-3061
- **KIPRIYANOVet al.** *J. Mol. Biol.*, 1999, vol. 293, 41-56
- **ROOVERS et al.** *Cancer Immunol.*, 2001
- **HOLLIGER P. et al.** *Proc. Natl. Acad. Sci. USA*, 1993, vol. 90, 6444-6448
- **POLJAK R. J. et al.** *Structure*, 1994, vol. 2, 1121-1123
- **KOHLER et al.** *Nature*, 1975, vol. 256, 495
- **MORRISON et al.** *Proc. Natl. Acad. Sci. USA*, 1984, vol. 81, 6851-6855
- **JONES et al.** *Nature*, 1986, vol. 321, 522-525
- **REICHMANN et al.** *Nature*, 1988, vol. 332, 323-329
- **PRESTA.** *Curr.Op.Struct.Biol.*, 1992, vol. 2, 593-596
- **CLARK.** *Immunol. Today*, 2000, vol. 21, 397-402
- Epitope Mapping Protocols in Methods in Molecular Biology. 1996, vol. 66
- Remington's Pharmaceutical Sciences. Mack Publishing Company, 1995
- **KABAT et al.** Sequences of Proteins of Immunological Interest. 1991, vol. 1-3
- **KABAT.** Sequences of Proteins of Immunological Interest. National Institutes of Health, 1991
- **AL-LAZIKANI et al.** Standard conformations for the canonical structures of immunoglobulins.. *J. Mol. Biol.*, 1997, vol. 273, 927-948
- **MARTIN AC et al.** Modeling antibody hypervariable loops: a combined algorithm. *Proc. Natl. Acid. Sci. USA*, 1989, vol. 86, 9268-9272
- **JOYCE F. LIU et al.** *JAMA Oncol.*, 2019, vol. 5 (12), 1731-1738
- **MANEGOLD C et al.** *J Thorac Oncol.*, February 2017, vol. 12 (2), 194-207
- **DUDEK AZ et al.** *J Clin Oncol.*, 2018, vol. 36
- **STEIN S et al.** *J Clin Oncol*, 2018, vol. 36 (15), 4074
- **HOCHSTER HS et al.** *American Society of Clinical Oncology Gastrointestinal Cancers Symposium*, 19 January 2017
- **YUKINORI OZAKI et al.** *AACR; Cancer Res*, 2020, vol. 80 (4)
- *J Immunother Cancer.*, 2024, vol. 12 (4), e008037
- *Immunity*, 2013, vol. 39 (1), 61-73

- *Cancer Metastasis Rev*, 2011, vol. 30 (1), 83-95
- *Clin Exp Immunol*, 2013, vol. 172 (3), 500-6
- **SZNOL et al.** *ASCO, GU*, 2015
- **HURWITZ et al.** *New Eng J Med*, 2004, vol. 350, 2335-2342
- **SANDLER et al.** *J Clin Oncol*, 2005, vol. 23 (16), 2